(19) **Europäisches Patentamt / European Patent Office / Office européen des brevets**

(11) **EP 4 241 657 A1**

(12) **EUROPEAN PATENT APPLICATION**

(43) Date of publication:
**13.09.2023 Bulletin 2023/37**

(21) Application number: **23160941.3**

(22) Date of filing: **09.03.2023**

(51) International Patent Classification (IPC):
**A61B 5/00** *(2006.01)* **A61B 5/0507** *(2021.01)*
**G16H 50/30** *(2018.01)*

(52) Cooperative Patent Classification (CPC):
**A61B 5/4815; A61B 5/0507; A61B 5/7264;**
**G16H 40/63; G16H 50/30;** A61B 5/0022;
A61B 5/0205; A61B 5/0826; A61B 5/1113;
A61B 5/113; A61B 5/1135; A61B 5/4818;
A61B 5/7282

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **10.03.2022 KR 20220030033**
**17.02.2023 KR 20230021306**

(71) Applicant: **Bitsensing Inc.**
**Seongnam-si, Gyeonggi-do 13105 (KR)**

(72) Inventor: **CHOE, Sun Taag**
**07013 Seoul (KR)**

(74) Representative: **BCKIP Part mbB**
**Siegfriedstraße 8**
**80803 München (DE)**

(54) **DEVICE, METHOD AND COMPUTER PROGRAM FOR SCORING SLEEP QUALITY**

(57) A device for scoring sleep quality includes a scoring function generation unit configured to generate distribution model for at least one of components of polysomnography based on polysomnography result information about a plurality of polysomnography testees and generate scoring function for the distribution model; a transceiver configured to transmit a radar signal toward a subject and receive the radar signal reflected from the subject; an information derivation unit configured to derive sleep time information about the subject and event occurrence information for a plurality of sleep items based on the radar signal; a component score calculation unit configured to calculate score for the at least one of components by applying the sleep time information and the event occurrence information to the scoring function; and a sleep quality calculation unit configured to calculate a sleep quality score of the subject based on the calculated score for the at least one of components.

*FIG. 19*

START

→ GENERATE DISTRIBUTION MODELS AND SCORING FUNCTIONS — S100

→ TRASNMIT AND RECEIVE RADAR SIGNAL — S200

→ DERIVE SLEEP TIME INFORMATION AND EVENT OCCURRENCE INFORMATION — S300

→ CALCULATE SCORES FOR RESPECTIVE COMPONENTS — S400

→ CALCULATE SLEEP QUALITY SCORE — S500

→ END

EP 4 241 657 A1

**Description**

**CROSS-REFERENCE TO RELATED APPLICATION**

This application claims the benefit under 35 use 119(a) of Korean Patent Applications No. 10-2022-0030033 filed on March 10, 2022, and No. 10-2023-0021306 filed on February 17, 2023 in the Korean Intellectual Property Office.

**TECHNICAL FIELD**

The present disclosure relates to a device, a method and a computer program for scoring sleep quality.

**BACKGROUND**

Polysomnography is a test for diagnosing a sleep disorder and is used to diagnose a sleep-related disorder and to determine a treatment method by collectively measuring brain waves, eye movement, body movement, breathing, electrocardiogram, etc. during sleep while videorecording a sleep state and then analyzing the measured and videorecorded data.

Polysomnography can diagnose symptoms such as sleep apnea, sleep disorder, sleepwalking, etc. As indexes for determining such diseases, a sleep stage, an apnea-hypopnea index (AHI), a respiratory disturbance index (RDI), etc. have been used.

Meanwhile, polysomnography employs a manual sleep scoring method that determines the above-described indexes by combining biometric data of a patient measured using various sensors by experts.

In other words, current polysomnography proceeds with sleep scoring based on a subjective determination standard of each expert. Therefore, the accuracy and reliability of a scoring result may be degraded.

Specifically, in polysomnography, although a determination standard for sleep scoring appears to be very clear, the form or magnitude of a biosignal differs for each patient and many errors occur even among experts due to irregularity of biosignals. In reality, it is known that when experts perform sleep scoring using the same biometric data of the same patient, a deviation in scoring is about 20%.

Sleep scoring is performed through a manual operation by an expert and thus requires a relatively large amount of time. In reality, it may take about 3 to 4 hours for a skilled expert to perform sleep scoring for a single patient.

Meanwhile, a basic method for measuring the quality and amount of sleep uses a wrist actigraphy device to measure sleep time. Specifically, sleep time of a wearer wearing a wrist actigraphy device is measured and tossing or the like during sleep is detected based on activities of the wearer. Also, a photoplethysmography (PPG) device worn on the wrist is used to measure the heart rate and heart rate variability of the wearer during sleep. Specifically, sleep stages of the wearer are distinguished, and oxygen desaturation caused by apnea is detected by measuring oxygen saturation $(SpO_2)$.

However, the conventional test method needs to be performed while a tester is worn on a part of a human body, and depends only on an expensive tester to more precisely examine sleep diseases.

Prior Art Document

(Patent Document 1) Korean Patent Publication No. 10-2321991 (registered on October 29, 2021)
(Patent Document 2) Korean Patent Laid-open Publication No. 10-2018-0077453 (published on July 9, 2018)

**SUMMARY**

In view of the foregoing, the present disclosure provides a device, a method and a computer program for scoring sleep quality that can analyze a sleep state of a human subject by using a radar and automatically score the quality of sleep based on the result of analysis to minimize the time required for measuring the quality of sleep.

Also, the present disclosure provides a device, a method and a computer program for scoring sleep quality that can determine the quality of sleep using various distribution models and scoring functions depending on the state of a testee to improve the accuracy and reliability of the derived sleep quality score.

The problems to be solved by the present disclosure are not limited to the above-described problems. There may be other problems to be solved by the present disclosure.

According to an exemplary embodiment, a device for scoring sleep quality may include a scoring function generation unit configured to generate distribution model for at least one of components of polysomnography based on polysomnography result information about a plurality of polysomnography testees and generate scoring function for the distribution model; a ransceiver configured to transmit a radar signal toward a subject and receive the radar signal reflected from the subject; an information derivation unit configured to derive sleep time information about the subject and event occurrence information for a plurality of sleep items based on the radar signal; a component score calculation unit configured to calculate score for the at least one of components by applying the sleep time information and the event occurrence information to the scoring function; and a sleep quality calculation unit configured to calculate a sleep quality score of the subject based on the calculated score for the at least one of components.

The above-described aspects are provided by way of illustration only and should not be construed as liming the present disclosure. Besides the above-described exemplary embodiments, there may be additional exemplary embodiments described in the accompanying drawings and the detailed description.

According to the present disclosure, it is possible to provide a device, a method and a computer program for scoring sleep quality that can analyze a sleep state of a human subject by using a radar and automatically score the quality of sleep based on the result of analysis to minimize the time required for measuring the quality of sleep.

Also, according to the present disclosure, it is possible to provide a device, a method and a computer program for scoring sleep quality that can determine the quality of sleep using various distribution models and scoring functions depending on the state of a testee to improve the accuracy and reliability of the derived sleep quality score.

## BRIEF DESCRIPTION OF THE DRAWINGS

In the detailed description that follows, embodiments are described as illustrations only since various changes and modifications will become apparent to a person with ordinary skill in the art from the following detailed description. The use of the same reference numbers in different figures indicates similar or identical items.

FIG. 1 is a configuration diagram of a sleep quality scoring system according to an embodiment of the present disclosure.

FIG. 2 is a configuration diagram of a sleep quality scoring device according to an embodiment of the present disclosure.

FIG. 3 is a diagram for explaining a process of calculating an average breathing signal according to an embodiment of the present disclosure.

FIG. 4 is a diagram for explaining a time sensitivity factor and an amplitude sensitivity factor according to an embodiment of the present disclosure.

FIG. 5 is a diagram for explaining first sleep breathing pattern information and second sleep breathing pattern information according to an embodiment of the present disclosure.

FIG. 6 is a diagram for explaining a first sleep breathing event and a second sleep breathing event according to an embodiment of the present disclosure.

FIG. 7A is a diagram for explaining optimal parameters for a plurality of sleep items according to an embodiment of the present disclosure.

FIG. 7B is a diagram showing an example of a data set for polysomnography according to an embodiment of the present disclosure.

FIG. 8 shows examples of distribution information of sleep-related symptoms according to an embodiment of the present disclosure.

FIG. 9 is a diagram for explaining a process of determining whether a subject has sleep-related symptoms according to an embodiment of the present disclosure.

FIG. 10 and FIG. 11 are diagrams for explaining a process of scoring sleep quality according to an embodiment of the present disclosure.

FIG. 12 and FIG. 13 show graphs for explaining distribution models according to an embodiment of the present disclosure.

**FIG. 14** and **FIG. 15** show graphs for explaining scoring functions according to an embodiment of the present disclosure.

**FIG. 16** is a graph for explaining a process of scoring sleep quality according to the scoring function of **FIG. 14.**

**FIG. 17** is provided to explain a process of calculating a sleep quality score based on scores calculated for respective components of polysomnography according to an embodiment of the present disclosure.

**FIG. 18** is provided to explain a process of calculating a sleep quality score based on weightings of scores calculated for respective components of polysomnography according to an embodiment of the present disclosure.

**FIG. 19** is a flowchart showing a method for scoring sleep quality according to an embodiment of the present disclosure.

**DETAILED DESCRIPTION**

Hereafter, example embodiments will be described in detail with reference to the accompanying drawings so that the present disclosure may be readily implemented by those skilled in the art.

However, it is to be noted that the present disclosure is not limited to the example embodiments but can be embodied in various other ways. In the drawings, parts irrelevant to the description are omitted for the simplicity of explanation, and like reference numerals denote like parts through the whole document.

Throughout this document, the term "connected to" may be used to designate a connection or coupling of one element to another element and includes both an element being "directly connected" another element and an element being "electronically connected" to another element via another element. Further, it is to be understood that the terms "comprises," "includes," "comprising," and/or "including" means that one or more other components, steps, operations, and/or elements are not excluded from the described and recited systems, devices, apparatuses, and methods unless context dictates otherwise; and is not intended to preclude the possibility that one or more other components, steps, operations, parts, or combinations thereof may exist or may be added. Throughout this document, when a member is said to be located "on" another member, this includes not only when the member is in contact with another member, but also when other member is present between the two members.

Throughout this document, the term "unit" may refer to a unit implemented by hardware, software, and/or a combination thereof. As examples only, one unit may be implemented by two or more pieces of hardware or two or more units may be implemented by one piece of hardware.

Throughout this document, a part of an operation or function described as being carried out by a terminal or device may be implemented or executed by a device connected to the terminal or device. Likewise, a part of an operation or function described as being implemented or executed by a device may be so implemented or executed by a terminal or device connected to the device.

Hereinafter, embodiments of the present disclosure will be explained in detail with reference to the accompanying drawings.

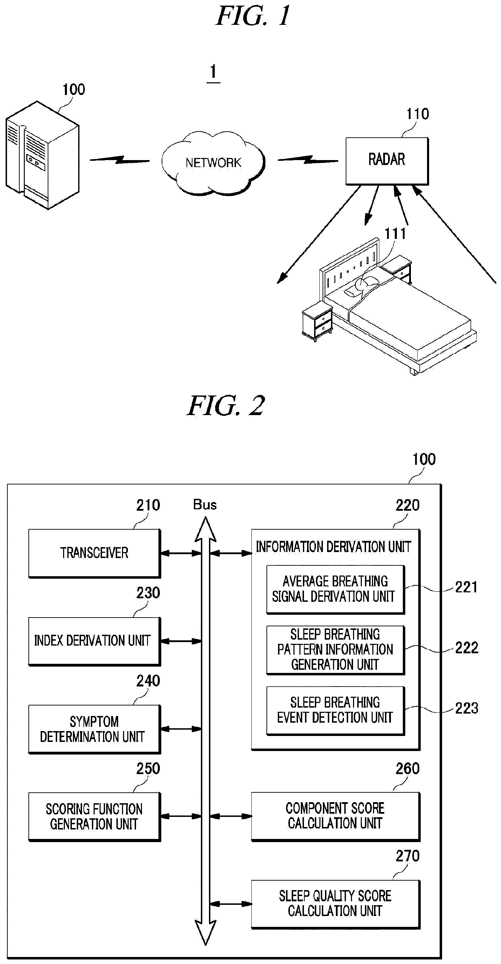

**FIG. 1** is a configuration diagram of a sleep quality scoring system according to an embodiment of the present disclosure.

Referring to **FIG. 1,** a sleep quality scoring system 1 may include a sleep quality scoring device 100 and a radar 110.

The components of the sleep quality scoring system 1 illustrated in **FIG. 1** are typically connected to each other via a network. For example, as illustrated in **FIG. 1,** the sleep quality scoring device 100 and the radar 110 may be connected simultaneously or sequentially. The network refers to a connection structure that enables information exchange between nodes such as devices, devices, etc. and includes LAN (Local Area Network), WAN (Wide Area Network), Internet (WWW: World Wide Web), a wired or wireless data communication network, a telecommunication network, a wired or wireless television network, and the like. Examples of the wireless data communication network may include 3G, 4G, 5G, 3GPP (3rd Generation Partnership Project), LTE (Long Term Evolution), WIMAX (World Interoperability for Microwave Access), Wi-Fi, Bluetooth communication, infrared communication, ultrasonic communication, VLC (Visible Light Communication), LiFi, and the like, but may not be limited thereto.

The sleep quality scoring device 100 may analyze breathing signals from a subject 111 by using the radar 110 during sleep of the subject 111. The sleep quality scoring device 100 may detect a sleep disorder of the subject 111 and determine a sleep-related disease by using the breathing signals of the subject 111.

For example, the sleep quality scoring device 100 is arranged to keep a predetermined distance from the subject 111 in sleep, and may transmit a radar signal toward the subject 111 and receive the radar signal reflected from the subject 111 by using the radar 110.

The sleep quality scoring device 100 may determine sleep-related symptoms, such as obstructive sleep apnea (OSA), central sleep apnea (CSA) and mixed sleep apnea, of the subject 111 by using the radar 110.

Therefore, the sleep quality scoring device 100 can precisely determine sleep-related symptoms of the subject 111 and analyze the causes as in polysomnography without performing polysomnography.

Since the sleep quality scoring device 100 analyzes the breathing signals related to the sleep of the subject 111 by using the radar 110, it is possible to reduce inconvenience incurred in performing polysomnography. Also, the sleep quality scoring device 100 can easily analyze sleep breathing of the subject 111 in daily life and continuously monitor sleep-related symptoms. Further, the sleep quality scoring device 100 can analyze sleep breathing of the subject 111 in a contactless manner.

Hereafter, each component of the sleep quality scoring device 100 will be described.

**FIG. 2** is a configuration diagram of a sleep quality scoring device according to an embodiment of the present disclosure. Referring to **FIG. 2,** the sleep quality scoring device 100 may include a transceiver 210, an information derivation unit 220, an index derivation unit 230, a symptom determination unit 240, a scoring function generation unit 250, a component score calculation unit 260 and a sleep quality score calculation unit 270. However, these components 210 to 270 are just examples of components that can be controlled by the sleep quality scoring device 100. The transceiver 210 may be understood as a component corresponding to the radar 110 of **FIG. 1.**

Hereafter, each component of the sleep quality scoring device 100 will be described.

The transceiver 210 may transmit a radar signal toward a subject. The transceiver 210 may receive the radar signal reflected from the subject. For example, the transceiver 210 may transmit a radar signal toward a subject and receive the radar signal reflected from the subject by using a radar.

The information derivation unit 220 may derive an average breathing signal of the subject by using the radar signal reflected from the subject, generate sleep breathing pattern information based on the average breathing signal, and detect a sleep breathing event occurring during sleep.

The information derivation unit 220 may include an average breathing signal derivation unit 221, a sleep breathing pattern information generation unit 222 and a sleep breathing event detection unit 223. The average breathing signal derivation unit 221 may derivate an average breathing signal of a subject based on a radar signal. For example, the average breathing signal derivation unit 221 may calculate a signal corresponding to usual breathing of the subject by using a pattern of the radar signal reflected from the subject and a change in amplitude of the signal.

The average breathing signal derivation unit 221 may calculate the average breathing signal of the subject by using the radar signal reflected from the subject and two sensitivity factors.

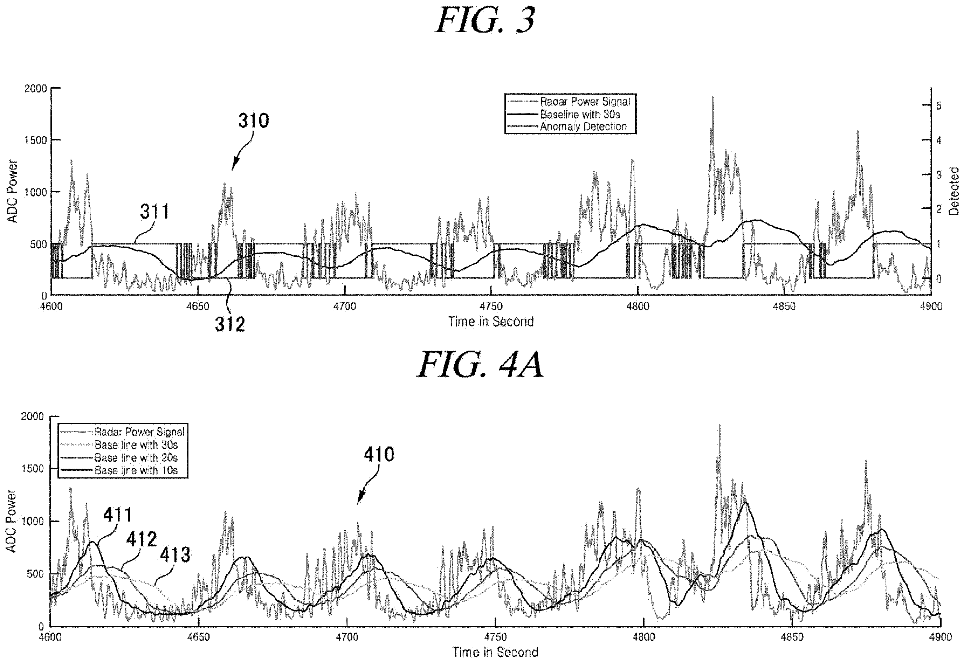

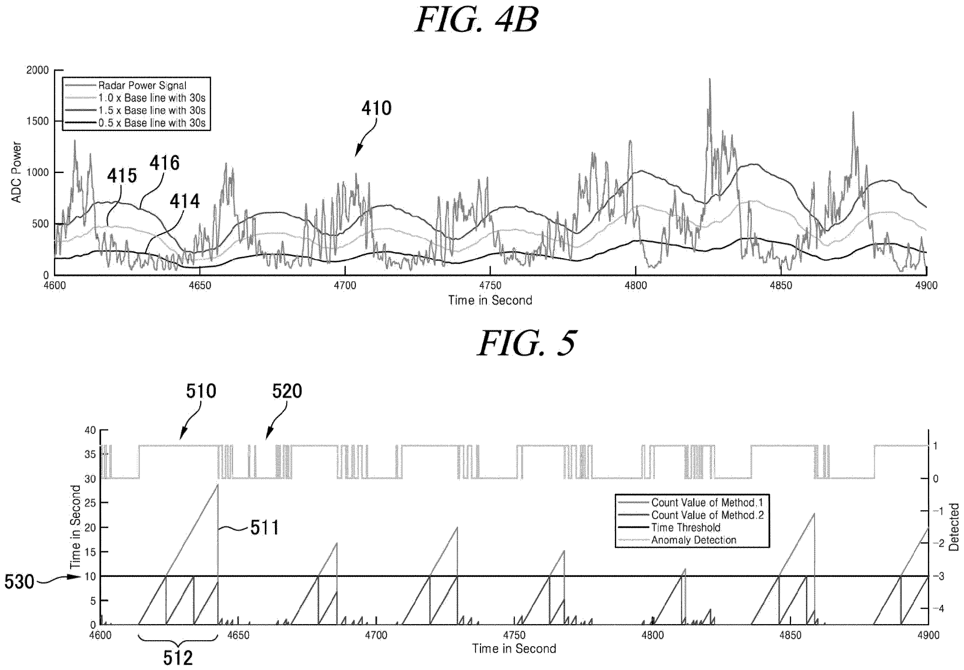

Hereafter, a process of calculating an average breathing signal will be described with reference to **FIG. 3** and **FIG. 4.**

**FIG. 3** is a diagram for explaining a process of calculating an average breathing signal according to an embodiment of the present disclosure. Referring to **FIG. 3,** the average breathing signal derivation unit 221 may derivate an average breathing signal 311 of the subject by using a radar signal 310 reflected from the subject and generate sleep breathing state information 312 of the subject.

For example, the average breathing signal derivation unit 221 may derivate the average breathing signal 311 by using a time sensitivity factor and an amplitude sensitivity factor based on the radar signal 310. The average breathing signal derivation unit 221 may use Equation 1 below.

$$<\text{Equation 1}> \quad m[i] = \frac{F}{L}\sum_{j=0}^{L-1} x[i-j]$$

In Equation 1, m an average breathing signal of a subject and x is the radar signal reflected and received from the subject. Also, I is a time sensitivity factor that means the length of time between specific consecutive periods and is an integer obtained by multiplying the frequency of the radar signal in sequence by the length of time in second. Further, F is an amplitude sensitivity factor that responds to the amplitude between the specific consecutive periods.

For example, the average breathing signal derivation unit 221 may calculate the average breathing signal of the subject by using the time sensitivity factor and the amplitude sensitivity factor based on the radar signal. Referring to Equation 1, the waveform of the average breathing signal of the subject may become obtuse over time as the time sensitivity factor increases and the amplitude sensitivity factor decreases, and may become sharp over time as the time sensitivity factor decreases and the amplitude sensitivity factor increases.

The average breathing signal derivation unit 221 may compare the radar signal 310 received from the subject with the average breathing signal 311. For example, the average breathing signal derivation unit 221 may detect a section in which the radar signal 310 is smaller than the average breathing signal 311. The average breathing signal derivation unit 221 may detect the section in which the radar signal 310 is smaller than the average breathing signal 311 and generate the sleep breathing state information 312.

The average breathing signal derivation unit 221 may assign "1" to the section in which the radar signal 310 is smaller than the average breathing signal 311 and "0" to the other section. For example, the average breathing signal derivation unit 221 may generate breathing state information about sleep breathing of the subject based on the detection result. That is, the sleep breathing state information 312 of the subject may be expressed by a value of "1" or "0".

**FIG. 4** is a diagram for explaining a time sensitivity factor and an amplitude sensitivity factor according to an embodiment of the present disclosure. For example, the average breathing signal derivation unit 221 may calculate an average breathing signal of a subject based on a radar signal received from the subject. In this case, the average breathing signal derivation unit 221 may use the time sensitivity factor and the amplitude sensitivity factor.

**FIG. 4A** is a graph showing an average breathing signal depending on a change in time sensitivity factor and derived from a radar signal 410, and **FIG. 4B** is a graph showing an average breathing signal depending on a change in amplitude sensitivity factor and derived from the radar signal 410.

Referring to the example shown in **FIG. 4A** and Equation 1 above, as a cycle of a time sensitivity factor decreases, the waveform of an average breathing signal corresponding to the time sensitivity factor may become sharp. For example, as a result of comparison among a waveform 411 of an average breathing signal corresponding to a time sensitivity factor with a cycle of 10 seconds, a waveform of an average breathing signal corresponding to a time sensitivity factor 412 with a cycle of 20 seconds and a waveform of an average breathing signal corresponding to a time sensitivity factor 413 with a cycle of 30 seconds, the waveform 411 of the average breathing signal corresponding to the time sensitivity factor with a cycle of 10 seconds changes most sharply.

Referring to the example shown in **FIG. 4B** and Equation 1 above, as a cycle of an amplitude sensitivity factor decreases, the waveform of an average breathing signal corresponding to the amplitude sensitivity factor may become obtuse. For example, as a result of comparison among a waveform 414 of an average breathing signal corresponding to an amplitude sensitivity factor with a cycle of 0.5, a waveform of an average breathing signal corresponding to an amplitude sensitivity factor 415 with a cycle of 1.0 and a waveform of an average breathing signal corresponding to an amplitude sensitivity factor 416 with a cycle of 1.5, the waveform 414 of the average breathing signal corresponding to the amplitude sensitivity factor with a cycle of 0.5 can be most obtuse.

The sleep breathing pattern information generation unit 222 may compare a radar signal with an average breathing signal and generate sleep breathing pattern information of a subject. For example, the sleep breathing pattern information generation unit 222 may detect a section in which a radar signal received from a subject is smaller than an average breathing signal of the subject.

The sleep breathing pattern information generation unit 222 may detect the section in which the radar signal is smaller than the average breathing signal and generate breathing state information about sleep breathing of the subject. That is, the sleep breathing pattern information generation unit 222 may generate sleep breathing state information of the subject that compares current sleep breathing of the subject with usual sleep breathing of the subject by using a radar. For example, the sleep breathing pattern information generation unit 222 may use Equation 2 below.

<Equation 2>

$$a_1[i] = \begin{cases} 1 & when\ x[i] < m[i] \\ 0 & otherwise \end{cases}$$

In Equation 2, $a_1$ is breathing state information about sleep breathing of a subject, $x[i]$ is the radar signal reflected and received from the subject, and $m[i]$ is an average breathing signal of the subject.

For example, the sleep breathing pattern information generation unit 222 may compare the radar signal received from the subject during sleep with the average breathing signal of the subject. The sleep breathing pattern information generation unit 222 may detect a section in which the radar signal received from the subject is smaller than the average breathing signal of the subject.

The sleep breathing pattern information generation unit 222 may generate first sleep breathing pattern information and second sleep breathing pattern information that represent the continuity of sleep state of the subject by using the breathing state information about sleep breathing of the subject.

Hereafter, a process of generating sleep breathing pattern information of a subject will be described with reference to **FIG. 5.**

**FIG. 5** is a diagram for explaining first sleep breathing pattern information and second sleep breathing pattern information according to an embodiment of the present disclosure. Referring to **FIG. 5,** the sleep breathing pattern information generation unit 222 may first generate an average breathing signal of a subject. For example, if a radar signal is smaller than an average breathing signal, current breathing state information of the subject may be derived as "1" (corresponding to reference numeral 510 in **FIG. 5**), and if the radar signal is greater than the average breathing signal, current breathing state information of the subject may be derived as "0" (corresponding to reference numeral 512 in **FIG. 5**).

Then, the sleep breathing pattern information generation unit 222 may compare the radar signal reflected from the subject with the average breathing signal of the subject and generate sleep breathing pattern information including first sleep breathing pattern information 511 and second sleep breathing pattern information 512.

The sleep breathing pattern information generation unit 222 may generate the first sleep breathing pattern information 511 of the subject based on whether the breathing state information continues. The sleep breathing pattern information generation unit 222 may generate the first sleep breathing pattern information 511 having a value that is increased when current sleep breathing of the subject is lower than usual sleep breathing. For example, the sleep breathing pattern information generation unit 222 may generate the first sleep breathing pattern information 511 by using Equation 3 below.

$$<\text{Equation 3}> \quad c_{11}[i] = \begin{cases} c_{11}[i-1] + 1 & when \ a_1[i] = 1 \ and \ a_1[i-1] = 1 \\ 0 & otherwise \end{cases}$$

In Equation 3, $c_{11}$ is the first sleep breathing pattern information 511. For example, referring to Equation 3 and **FIG. 5,** the sleep breathing pattern information generation unit 222 may calculate the first sleep breathing pattern information 511 having a value that is increased when the radar signal reflected from the subject, i.e., current breathing of the subject, is smaller than the average breathing signal of the subject and is maintained. That is, the first sleep breathing pattern information 511 may have a value that is increased when current breathing state information of the subject is maintained at "1" (corresponding to reference numeral 510 in **FIG. 5**) and may refer to the entire section in which the value is smaller than the average breathing signal during sleep of the subject.

For another example, referring to Equation 3 and **FIG. 5,** the sleep breathing pattern information generation unit 222 may initialize the first sleep breathing pattern information 511 when current breathing of the subject is greater than the average breathing signal of the subject. That is, the first sleep breathing pattern information 511 may be initialized when the breathing state information of the subject is not "1" (corresponding to reference numeral 520 in **FIG. 5**).

The sleep breathing pattern information generation unit 222 may generate the second sleep breathing pattern information 512 based on whether the breathing state information continues and a predetermined threshold value. The sleep breathing pattern information generation unit 222 may generate the second sleep breathing pattern information 512 having a value that is increased when current sleep breathing of the subject is lower than usual sleep breathing, initialized at a specific threshold time and then increased again. For example, the sleep breathing pattern information generation unit 222 may generate the second sleep breathing pattern information 512 by using Equation 4 below.

$$<\text{Equation 4}>$$

$$c_{12}[i] = \begin{cases} c_{12}[i-1] + 1 & when \ a_1[i] = 1 \ and \ a_1[i-1] = 1 \\ & and \ c_{12}[i-1] < T \\ 0 & otherwise \end{cases}$$

In Equation 4, $c_{12}$ is the second sleep breathing pattern information 512 and T is a specific threshold time 530 as a predetermined threshold value. For example, referring to Equation 4 and **FIG. 5,** the sleep breathing pattern information generation unit 222 may calculate the second sleep breathing pattern information 512 having a value that is increased when the radar signal reflected from the subject, i.e., current breathing of the subject, is smaller than the average breathing signal of the subject and is maintained like the first sleep breathing pattern information 511, but is initialized at the specific threshold time 530 (T in Equation 4) and then increased again.

For example, referring to Equation 4 and **FIG. 5,** the sleep breathing pattern information generation unit 222 may increase the value of the second sleep breathing pattern information 512 when current breathing state information of the subject is maintained at "1" 510, and may initialize the value of the second sleep breathing pattern information 512 at the specific threshold time 530 and then increase the value again.

Also, the sleep breathing pattern information generation unit 222 may detect a case where a radar signal is greater than an average breathing signal and generate motion state information about sleep breathing of the subject. For example, the sleep breathing pattern information generation unit 222 may generate first sleep motion pattern information and second sleep motion pattern information that represent the continuity of sleep motion state of the subject by using the motion state information about a motion detected during sleep of the subject.

First, the sleep breathing pattern information generation unit 222 may detect a case where the radar signal is greater than the average breathing signal and generate motion state information about sleep breathing of the subject.

That is, the sleep breathing pattern information generation unit 222 may generate sleep motion state information of the subject that compares current sleep breathing of the subject with usual sleep breathing of the subject by using a radar. For example, the sleep breathing pattern information generation unit 222 may use Equation 5 below.

<Equation 5>

$$a_2[i] = \begin{cases} 1 & when \ x[i] > m[i] \\ 0 & otherwise \end{cases}$$

In Equation 5, $a_2$ is motion state information about sleep breathing of a subject, x[i] is the radar signal reflected and received from the subject, and m[i] is an average breathing signal of the subject.

For example, referring to Equation 5, the sleep breathing pattern information generation unit 222 may detect a section in which the radar signal received from the subject during sleep is greater than the average breathing signal of the subject and generate motion state information about sleep breathing of the subject, first sleep motion pattern information and second sleep motion pattern information based on the detection result.

The sleep breathing pattern information generation unit 222 may generate the first sleep motion pattern information about a sleep motion of the subject based on whether the motion state information continues. The sleep breathing pattern information generation unit 222 may generate the first sleep motion pattern information having a value that is increased when current sleep breathing of the subject is higher than usual sleep breathing. For example, the sleep breathing pattern information generation unit 222 may use Equation 6 below.

<Equation 6>

$$c_{21}[i] = \begin{cases} c_{21}[i-1] + 1 & when \ a_2[i] = 1 \ and \ a_2[i-1] = 1 \\ 0 & otherwise \end{cases}$$

In Equation 6, $c_{21}$ is first sleep motion pattern information and $a_2$ is motion state information about sleep breathing of a subject. For example, referring to Equation 6, the sleep breathing pattern information generation unit 222 may calculate the first sleep motion pattern information having a value that is increased when the radar signal reflected from the subject is greater than the average breathing signal of the subject and is maintained. That is, the first sleep motion pattern information may refer to the entire section in which the value is greater than the average breathing signal during sleep of the subject.

The sleep breathing pattern information generation unit 222 may generate second sleep motion pattern information based on whether the motion state information continues and a predetermined threshold value. The sleep breathing pattern information generation unit 222 may generate the second sleep motion pattern information having a value that is increased when current sleep breathing of the subject is higher than usual sleep breathing, initialized at a specific threshold time and then increased again. For example, the sleep breathing pattern information generation unit 222 may use Equation 7 below.

<Equation 7>

$$c_{22}[i] = \begin{cases} c_{22}[i-1] + 1 & \begin{matrix} when \ a_2[i] = 1 \ and \ a_2[i-1] = 1 \\ and \ c_{22}[i-1] < T \end{matrix} \\ 0 & otherwise \end{cases}$$

In Equation 7, $c_{22}$ is second sleep motion pattern information and T is a specific threshold time as a predetermined threshold value. For example, referring to Equation 7, the sleep breathing pattern information generation unit 222 may calculate the second sleep motion pattern information having a value that is increased when the radar signal reflected from the subject is greater than the average breathing signal of the subject and is maintained like the first sleep motion pattern information, but is initialized at the specific threshold time and then increased again.

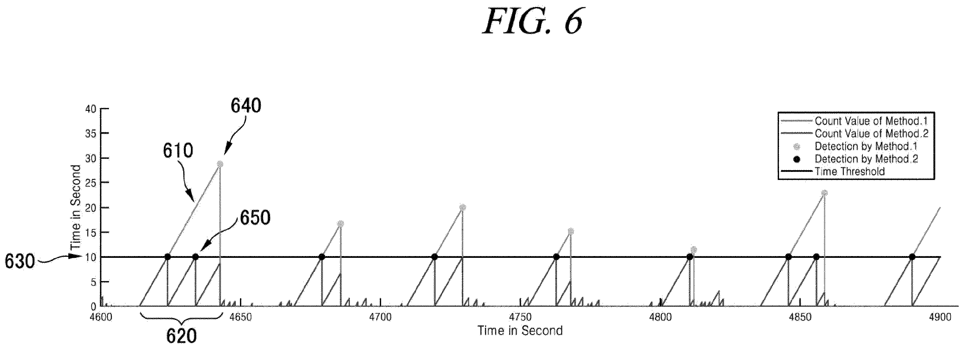

The sleep breathing event detection unit 223 may detect a sleep breathing event based on the sleep breathing pattern information. For example, the sleep breathing event detection unit 223 may detect a first sleep breathing event and a second sleep breathing event during sleep of the subject by using the first sleep breathing pattern information and the second sleep breathing pattern information. Hereafter, a process of generating sleep breathing pattern information of a subject will be described with reference to **FIG. 6.**

**FIG. 6** is a diagram for explaining a first sleep breathing event and a second sleep breathing event according to an embodiment of the present disclosure. Referring to **FIG. 6,** the sleep quality scoring device 100 may detect a first sleep breathing event and a second sleep breathing event by detecting peaks 640 and 650 from first sleep breathing pattern information 610 and second sleep breathing pattern information 620.

The sleep breathing event detection unit 223 may detect the first sleep breathing event based on a value corresponding to a threshold value or more in the first sleep breathing pattern information 610. The sleep breathing event detection unit 223 may detect, as the first sleep breathing event, the number of breathings when sleep breathing of the subject is lower than usual in a period longer than a specific threshold time during sleep of the subject. For example, the sleep breathing event detection unit 223 may detect the first sleep breathing event by using Equation 8 below.

<Equation 8>

$$d_{11}[i] = \begin{cases} 1 & when \ c_{11}[i-1] \geq T \ and \ c_{11}[i-1] > c_{11}[i] \\ 0 & otherwise \end{cases}$$

In Equation 8, $d_{11}$ is a first sleep breathing event, $c_{11}$ is the first sleep breathing pattern information 610, and T is a specific threshold time 630 as a predetermined threshold value. Referring to Equation 8 and **FIG. 6,** the sleep quality scoring device 100 may detect and count the peak 640 from the first sleep breathing pattern information 610 in a period longer than the specific threshold time 630 and thus detect the number of first sleep breathing events.

The sleep breathing event detection unit 223 may detect the second sleep breathing event based on a value reaching the threshold value in the second sleep breathing pattern information 620. The sleep breathing event detection unit 223 may detect, as the second sleep breathing event, the number of sleep breathings reaching the specific threshold time during sleep of the subject. For example, the sleep quality scoring device 100 may detect the second sleep breathing event by using Equation 9 below.

<Equation 9>

$$d_{12}[i] = \begin{cases} 1 & when \ c_{12}[i] = T \\ 0 & otherwise \end{cases}$$

In Equation 9, $d_{12}$ is a second sleep breathing event, $c_{12}$ is the second sleep breathing pattern information 620, and T is the specific threshold time 630 as a predetermined threshold value. Referring to Equation 9 and **FIG. 6,** the sleep quality scoring device 100 may detect and count the peak 650, which reaches the specific threshold time 630, from the second sleep breathing pattern information 620 and thus detect the number of second sleep breathing events.

That is, the sleep breathing event detection unit 223 may express a pattern depending on the duration of an apnea/hypopnea event by detecting the first sleep breathing event and the second sleep breathing event in a mixed manner.

Also, the sleep breathing event detection unit 223 may detect a sleep motion event based on sleep motion pattern information. The sleep breathing event detection unit 223 may detect a first sleep motion event based on a value corresponding to a threshold value or more in the first sleep motion pattern information. The sleep breathing event detection unit 223 may detect, as the first sleep motion event, the number of motions when sleep breathing of the subject is higher than usual in a period longer than a specific threshold time during sleep of the subject.

For example, the sleep breathing event detection unit 223 may use Equation 10 below.

<Equation 10>

$$d_{21}[i] = \begin{cases} 1 & when \ c_{21}[i-1] \geq T \ and \ c_{21}[i-1] > c_{21}[i] \\ 0 & otherwise \end{cases}$$

In Equation 10, $d_{21}$ is a first sleep motion event, $c_{21}$ is a first sleep motion pattern information, and T is a specific threshold time as a predetermined threshold value. Referring to Equation 10, the sleep breathing event detection unit 223 may detect and count a peak from the first sleep motion pattern information in a period longer than the specific threshold time and thus detect the number of first sleep motion events.

The sleep breathing event detection unit 223 may detect a second sleep motion event based on a value reaching the threshold value in the second sleep motion pattern information. The sleep breathing event detection unit 223 may detect, as the second sleep motion event, the number of sleep motions reaching the specific threshold time during sleep of the subject. For example, the sleep breathing event detection unit 223 may detect the second sleep motion event by using Equation 11 below.

<Equation 11>

$$d_{22}[i] = \begin{cases} 1 & when\ c_{22}[i] = T \\ 0 & otherwise \end{cases}$$

In Equation 11, $d_{22}$ is a second sleep motion event, $c_{22}$ is a second sleep motion pattern information, and T is a specific threshold time as a predetermined threshold value. Referring to Equation 11, the sleep breathing event detection unit 223 may detect and count a peak, which reaches the specific threshold time, from the second sleep motion pattern information and thus detect the number of second sleep motion events.

That is, the sleep breathing event detection unit 223 may suppress a detection error of a sleep breathing event by detecting the first sleep motion event and the second sleep motion event in a mixed manner.

The sleep breathing event detection unit 223 may set optimal parameters for a plurality of sleep items (or sleep test items). For example, the sleep breathing event detection unit 223 may set sleep test items that can be extracted by using a radar. The sleep breathing event detection unit 223 may set various sleep test items including apnea, hypopnea, central sleep apnea that causes apnea and hypopnea, and various temporary arousal and sleep stages based on human abdominal and limb motions.

The optimal parameters may include event occurrence-related parameters and signal processing parameters. For example, the event occurrence-related parameters may be regression coefficients for the first sleep breathing pattern information, the second sleep breathing pattern information, the first sleep motion pattern information and the second sleep motion pattern information detected during sleep of the subject, and the signal processing parameters correspond to, for example, F, T and L of Equation 1 and may be parameters for detecting each of the plurality of sleep items.

The sleep breathing event detection unit 223 may detect event occurrence information for the plurality of sleep items based on the optimal parameters. For example, the sleep breathing event detection unit 223 may calculate the number of events for each of the plurality of sleep items by varying the signal processing parameters.

For example, the sleep breathing event detection unit 223 may detect event occurrence information for the plurality of sleep items by using Equation 12 below.

<Equation 12>

$$Y = b_0 + b_1 N_{11} + b_2 N_{12} + b_3 N_{21} + b_4 N_{22} + e$$

For example, in Equation 12, Y may be the total number of events corresponding to event occurrence information, and $b_0$ to $b_4$ may be calculated by using a least squares method as event occurrence-related parameters. $N_{11}$ to $N_{22}$ are the numbers of events respectively corresponding to a first sleep breathing event, a second sleep breathing event, a first sleep motion event and a second sleep motion event detected by the above-described methods, and e may be an error term.

For example, the sleep breathing event detection unit 223 may detect a first sleep breathing event, a second sleep breathing event, a first sleep motion event and a second sleep motion event for an entry Central based on the signal processing parameters F, L and T previously set for the entry Central related to apnea, and may detect event occurrence information for the entry Central related to apnea by substituting the preset event occurrence-related parameters and the total number of first sleep breathing events, the total number of second sleep breathing events, the total number of first sleep motion events, and the total number of second sleep motion events detected for the entry Central into Equation 12.

The index derivation unit 230 may derive breathing-related index information based on sleep time information and the event occurrence information for the plurality of sleep items. For example, the sleep time information may correspond to the total sleep time of a test subject. The index derivation unit 230 may derive the breathing-related index information by dividing the total number of event occurrence information calculated for the plurality of sleep items by the sleep time information.

The index derivation unit 230 may calculate breathing-related index information including apnea-hypopnea index information and RDI information. The index derivation unit 230 may derive the apnea-hypopnea index information based on event occurrence information for an entry Apnea among the plurality of sleep items and event occurrence information related to an entry Hypopnea. For example, the index derivation unit 230 may use Equation 13 below.

<Equation 13>

$$AHI = \frac{\sum \text{Number of apneas} + \sum \text{Number of hypopneas}}{\text{Total sleep time}}$$

In Equation 13, AHI is apnea-hypopnea index (AHI) information, $\Sigma$ number of apneas is the total sum of event occurrence information for detection items detected related to apnea, and $\Sigma$ number of hypopneas is the total sum of event occurrence information for detection items detected related to hypopnea.

For example, referring to Equation 13, the index derivation unit 230 may calculate the number of apneas by summing up event occurrence information for an entry Central, event occurrence information for an entry Mixed and event occurrence information for an entry Obstructive detected related to apnea which are detected related to apnea, and may calculate the number of hypopneas by summing up event occurrence information for an entry Central, event occurrence information for an entry Mixed and event occurrence information for an entry Obstructive which are detected related to hypopnea.

For example, the index derivation unit 230 may predict a sleep time of a subject by motion analysis based on a radar signal received from the subject. The index derivation unit 230 may calculate the apnea-hypopnea index information by dividing the number of apneas and the number of hypopneas by the total sleep time of the subject.

The index derivation unit 230 may derive the RDI information based on the event occurrence information for the entry Apnea among the plurality of sleep items, the event occurrence information for the entry Hypopnea and event occurrence information for an entry Respiratory Effort. For example, the index derivation unit 230 may use Equation 14 below.

<Equation 14>

$$RDI = \frac{\sum \text{Number of apneas} + \sum \text{Number of hypopneas} + \sum \text{Number of Respiratory Effort Related Arousal}}{\text{Total sleep time}}$$

In Equation 14, RDI is respiratory disturbance index (RDI) information, and $\Sigma$ number of apneas , $\Sigma$ number of hypopneas and $\Sigma$Number of respiratory effort related arousal may be the total sums of event occurrence information for detection items detected related to apnea, hypopnea and respiratory effort related arousal, respectively.

For example, referring to Equation 14, the index derivation unit 230 may calculate the RDI information by dividing the number of apneas, the number of hypopneas and the number of respiratory effort related arousals detected from a radar signal received from the subject by the total sleep time of the subject.

The symptom determination unit 240 may determine whether a symptom is included in sleep-related symptoms based on the breathing-related index information. The symptom determination unit 240 may compare a disease group distribution information of sleep-related symptoms and a control group distribution information with the breathing-related index information to determine whether a symptom is included in the sleep-related symptoms. For example, the symptom determination unit 240 may compare the disease group distribution information of sleep-related symptoms and the control group distribution information with the breathing-related index information by using a deep learning model. In this case, a disease group and a control group of the present disclosure may include a disease group with Alzheimer's dementia and a control group.

For example, the symptom determination unit 240 may use distribution values of sleep components of the disease group with sleep-related symptoms and distribution values of sleep components of the control group. For example, the symptom determination unit 240 may use the mean and standard deviation as the distribution values of sleep components of the disease group and control group. The symptom determination unit 240 may compare the apnea-hypopnea index information with the mean and standard deviation of the disease group with related symptoms and compare the apnea-hypopnea index information with the mean and standard deviation of the control group.

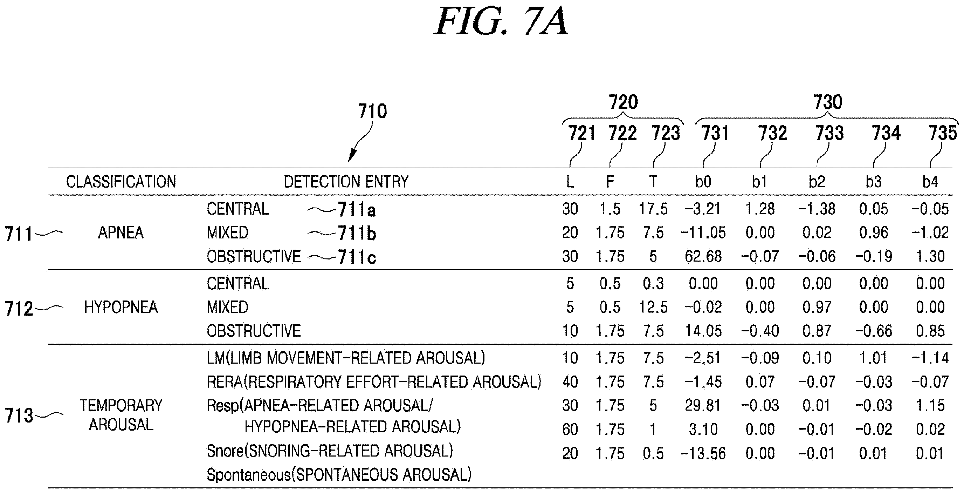

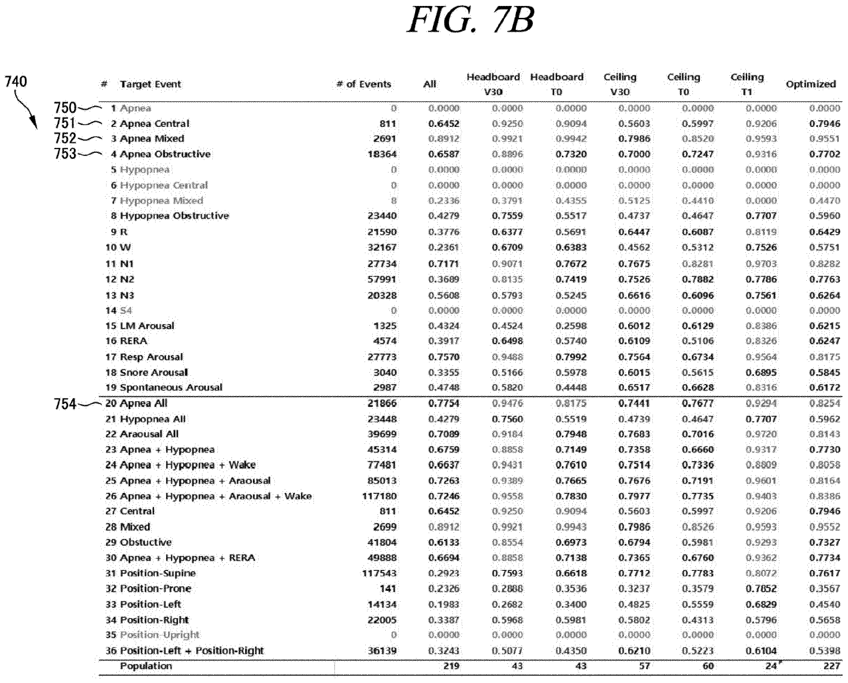

For example, the symptom determination unit 240 may determine whether the subject has sleep-related symptoms based on the comparison result. The symptom determination unit 240 may compare the apnea-hypopnea index information of the subject with distribution information of a disease group with apnea or distribution information of a control group and determine whether the subject has sleep apnea based on the comparison result. **FIG. 7A** is a diagram for explaining optimal parameters for a plurality of sleep items according to an embodiment of the present disclosure, and **FIG. 7B** is a diagram showing an example of a data set for polysomnography according to an embodiment of the present disclosure.

Referring to **FIG. 7A** and **FIG. 7B,** the sleep quality scoring device 100 may set a plurality of sleep items 710 that can be detected by using a radar and set optimal parameters for the respective items. The sleep quality scoring device 100 may detect event occurrence information based on the optimal parameters set for the plurality of sleep items 710.

First, as shown in **FIG. 7A,** the sleep quality scoring device 100 may set detection items 710 for each of a plurality of sleep items related to Apnea 711, Hypopnea 712 and Arousal 713. The sleep quality scoring device 100 may set an entry Central 711a, an entry Mixed 711b and an entry Obstructive 711c, which cause Apnea 711, as the detection items 710 for Apnea 711, set an entry Central, an entry Mixed and an entry Obstructive, which cause Hypopnea 712, as the detection items 710 for Hypopnea 712, and set an entry Limb Movement (LM), an entry Respiratory Effort Related Arousal (RERA), an entry Respiratory (Resp), an entry Snore and an entry Spontaneous, which cause Arousal 713, as the detection items 710 for Arousal 713.

For example, the sleep quality scoring device 100 may set optimal parameters including signal processing parameters 720 and event occurrence-related parameters 730 for the detection items 710 set for each of the plurality of sleep items.

As shown in **FIG. 7A,** the sleep quality scoring device 100 may set the signal processing parameters 720 including a time sensitivity factor that means the length of time between specific consecutive periods, L 721, an amplitude sensitivity factor that responds to the amplitude between the specific consecutive periods, F 722, a specific threshold time and T 723.

The sleep quality scoring device 100 may set the event occurrence-related parameters 730 including a regression coefficient $b_1$ 732 for a first sleep breathing event, a regression coefficient $b_2$ 733 for a second sleep breathing event, a regression coefficient $b_3$ 734 for a first sleep motion event, a regression coefficient $b_4$ 735 for a second sleep motion event, other regression coefficients $b_0$ 731 which area detected from the radar signal reflected from the subject.

For example, the sleep quality scoring device 100 may calculate event occurrence information by varying the signal processing parameters 720 for the plurality of sleep items and derive an optimal factor and a regression coefficient for each sleep entry by regression analysis. For example, L 721 may be one of 10s, 20s, 30s, 40s, 50s and 60s, F 722 may be one of 0.5, 0.75, 1, 1.25, 1.5, 1.75 and 2, and T 723 may be one of 5s, 7.5s, 10s, 12.5s, 15s, 17.5s and 20s to vary the signal processing parameters 720.

Referring to **FIG. 7B,** the sleep quality scoring device 100 may set optimal parameters for the plurality of sleep items based on a data set 740 for polysomnography.

For example, the sleep quality scoring device 100 may set optimal parameters including the signal processing parameters 720 and the event occurrence-related parameters 730 for the entry Central 711a related to Apnea 711 based on the data set 740 shown in **FIG. 7B.**

For example, referring to **FIG. 7A** and **FIG. 7B,** the sleep quality scoring device 100 may set the signal processing parameters 720 and the event occurrence-related parameters 730 for the entry Central 711a related to Apnea 711 based on information about an entry Apnea Central 751 that causes Apnea 750 measured from the data set 740 by polysomnography, and may set the signal processing parameters 720 and the event occurrence-related parameters 730 for the entry Mixed 711b related to Apnea 711 based on information about an entry Apnea Mixed 752 that causes Apnea 750 measured by polysomnography.

Further, the sleep quality scoring device 100 may set the signal processing parameters 720 and the event occurrence-related parameters 730 for Apnea 711 based on information about Apnea All 754 measured by polysomnography.

For example, referring to **FIG. 7A** and **FIG. 7B,** the sleep quality scoring device 100 may determine a factor with the highest correlation coefficient with a correct answer value measured by polysomnography as an optimal factor and thus may set the signal processing parameters 720 including the detection items 710 for Apnea 711, the time sensitivity factor, L 721 of "30s", the amplitude sensitivity factor, F 722 of "1.5", the specific threshold time and T 723 of "17.5" for the entry Central 711a as optimal parameters with reference to the data set 740, and may set the event occurrence-related parameters 730 including the regression coefficients, $b_0$ 731 of "-3.21", $b_1$ 732 of "1.28", $b_2$ 733 of "-1.38", $b_3$ 734 of "0.05" and $b_4$ 735 of "-0.05".

For example, the sleep quality scoring device 100 may derive event occurrence information for the plurality of sleep items based on the optimal parameters set for the plurality of sleep items (see Equation 12 above). Specifically, the sleep quality scoring device 100 may derive event occurrence information for the entry Central related to apnea of the subject by using information detected related to the entry Central 711a of Apnea 711, the first sleep breathing event, the second sleep breathing event, the first sleep motion event and the second sleep motion event, and the optimal parameters 720 and 730 set for the entry Central 711a of Apnea 711.

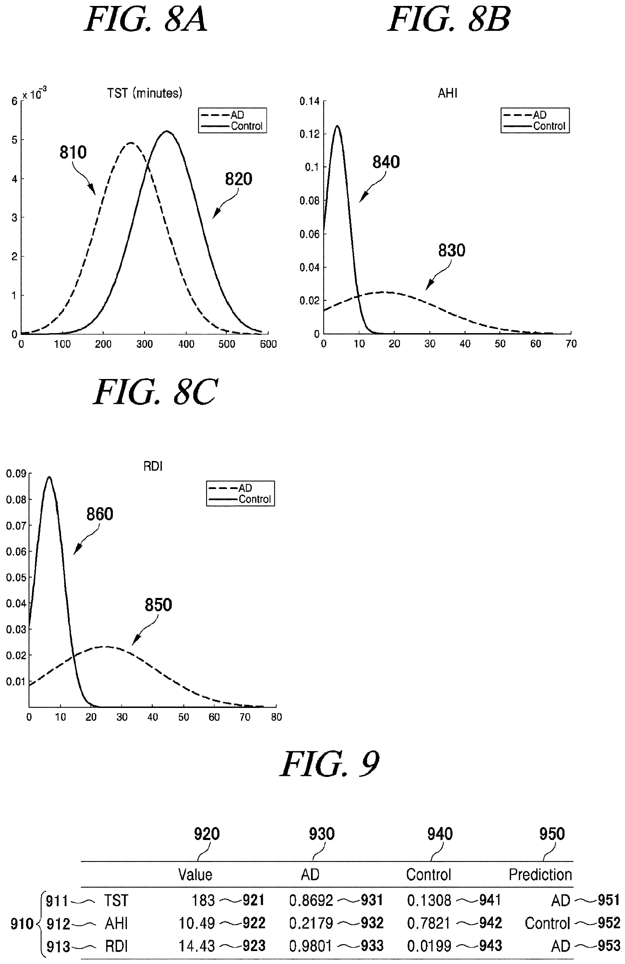

**FIG. 8** shows examples of distribution information of sleep-related symptoms of according to an embodiment of the present disclosure. **FIG. 8A** shows distribution information of total sleep time TST of a disease group with Alzheimer's dementia and a control group, **FIG. 8B** shows distribution information of apnea-hypopnea of the disease group with Alzheimer's dementia and the control group, and **FIG. 8C** shows distribution information of respiratory disturbance of the disease group with Alzheimer's dementia and the control group. The distribution information shown in **FIG. 8A** to **FIG. 8C** may be graphed by processing data about the total sleep time, apnea-hypopnea index and RDI of Alzheimer's dementia, and may include mean and standard deviation.

For example, the sleep quality scoring device may compare the distribution information of sleep-related symptoms and determine whether the subject is included in the disease group with the corresponding sleep-related symptoms.

For example, referring to **FIG. 8A,** the sleep quality scoring device 100 may compare the total sleep time of the subject with distribution information 810 of the total sleep time of the disease group with Alzheimer's dementia, and may compare the total sleep time of the subject with distribution information 820 of the total sleep time of the control group. The sleep quality scoring device 100 may determine whether distribution information of the total sleep time of the subject is included in the disease group with Alzheimer's dementia based on the comparison result.

For example, referring to **FIG. 8B,** the sleep quality scoring device 100 may compare the apnea-hypopnea index information detected from the subject with distribution information 830 of apnea-hypopnea of the disease group with Alzheimer's dementia, and may compare the apnea-hypopnea index information of the subject with distribution information 840 of apnea-hypopnea of the control group. The sleep quality scoring device 100 may determine whether the apnea-hypopnea index information of the subject is included in the disease group with Alzheimer's dementia based on the comparison result.

For example, referring to **FIG. 8C,** the sleep quality scoring device 100 may compare RDI information detected from the subject with distribution information 850 of respiratory disturbance of the disease group with Alzheimer's dementia, and may compare the RDI information of the subject with distribution information 860 of respiratory disturbance of the control group. The sleep quality scoring device 100 may determine whether the RDI information of the subject is included in the disease group with Alzheimer's dementia based on the comparison result.

**FIG. 9** is a diagram for explaining a process of determining whether a subject has sleep-related symptoms according to an embodiment of the present disclosure. Referring to **FIG. 9,** the sleep quality scoring device 100 may compare breathing-related index information 920 derived from the subject for a plurality of breathing-related index items 910 with each of distribution information of a disease group with sleep-related symptoms and distribution information of a control group, and may generate prediction information 950 by determining whether the subject has sleep-related symptoms in terms of probability distribution based on the comparison result.

For example, the sleep quality scoring device 100 may compare the index information 920 of the subject for the plurality of breathing-related index items 910 with the distribution information of the disease group with sleep-related symptoms to derive information 930 about the probability of the subject being included in the disease group, and may compare the index information 920 of the subject for the plurality of breathing-related index items 910 with the distribution information of the control group to derive information 940 about the probability of the subject being included in a normal group. The sleep quality scoring device 100 may generate the prediction information 950 by determining whether the subject is included in the disease group with sleep-related symptoms based on the derived probability information 930 and 940.

Referring to the example shown in **FIG. 9,** the sleep quality scoring device 100 may derive information "0.8692" 931 about the probability of being included in the disease group and information "0.1308" 941 about the probability of being included in the control group based on the result of comparing index information "183" 921 of total sleep time 911 of the subject with the distribution information of the total sleep time of the disease group and the distribution information of the total sleep time of the control group. Accordingly, the sleep quality scoring device 100 may generate prediction information "AD" 951 by determining that the subject is included in the disease group with sleep-related symptoms in terms of probability distribution based on the total sleep time of the subject.

The sleep quality scoring device 100 may derive information "0.2179" 932 about the probability of being included in the disease group and information "0.7821" 942 about the probability of being included in the control group based on the result of comparing index information "10.49" 922 of an apnea-hypopnea index 912 of the subject with the distribution information of apnea-hypopnea of the disease group and the distribution information of apnea-hypopnea of the control group. Accordingly, the sleep quality scoring device 100 may generate prediction information "Control" 952 by determining that the subject is included in the normal group in terms of probability distribution based on the apnea-hypopnea index information of the subject.

The sleep quality scoring device 100 may derive information "0.9801" 933 about the probability of being included in the disease group and information "0.0199" 943 about the probability of being included in the control group based on the result of comparing index information "14.43" 923 of RDI 913 of the subject with the distribution information of respiratory disturbance of the disease group and the distribution information of respiratory disturbance of the control group. Accordingly, the sleep quality scoring device 100 may generate prediction information "AD" 953 by determining that the subject is included in the disease group in terms of probability distribution based on the RDI information of the subject.

In this case, a final determination result as to the disease group/control group for each entry may be determined by voting. For example, the total sleep time 911 and the RDI 913 of the subject are determined as being included in the disease group and the apnea-hypopnea index 912 is determined as being included in the normal group, and, thus, the subject is predominantly determined as being included in the disease group for each entry. Therefore, the subject is finally determined as being included in the disease group and symptoms are determined.

As described above, according to the present disclosure, it is possible to determine a symptom by using the result of analysis of a radar signal during sleep of a subject, and also possible to provide a symptom determination service for providing the subject with information about the degermation of symptom.

Besides, information that becomes a basis for determination may be gathered after the degermation of symptom. The gathered information may also provide a basis for determination of symptom to the user at the time of service.

The location of the subject and the probability may be expressed on a specific domain information by refining the items simply used for test and criteria for determination of symptom for each entry.

Also, more detailed information may be derived and may be provided to the subject. The testee (subject) is included in the normal group in terms of the apnea-hypopnea index (AHI) 912, but is determined as being included in the disease group with a very high probability in terms of the RDI 913 to which the average number of RERAs is added. That is, the number of RERAs contributes greatly to an increase in the RDI, and the RERA is very related to central sleep apnea. Therefore, the results of items related to "entry Central" among a plurality of sleep test items may be provided together to the subject.

When data on various cases are accumulated later in addition to the above-described information, statistical information such as disease location in a similar age group, a similar home environment group and a similar underlying disease group may be provided together.

Hereafter, a process of generating distribution models and scoring functions based on polysomnography result information, substituting information generated from radar signals into the scoring functions and deriving a sleep quality score by the sleep quality scoring device 100 will be described.

Although not illustrated in **FIG. 1,** an external polysomnography device (not shown) is connected to the sleep quality scoring device 100, and the sleep quality scoring device 100 may receive polysomnography result information from the polysomnography device.

Herein, the polysomnography device refers to a device that performs polysomnography and measures biometric data, such as brain waves, eye movement, body movement, breathing and electrocardiogram, from a polysomnography testee during sleep to diagnose a sleep disorder.

The polysomnography device may generate biometric data of a sleeping user measured by using at least one sensor among an electroencephalogram (EEG) sensor, an electrooculography (EOG) sensor, an electromyogram (EMG) sensor, an electrocardiogram (EKG) sensor, a photoplethysmography (PPG) sensor, a chest belt, an abdomen belt, a thermistor, a flow sensor, and a microphone.

That is, the polysomnography device may generate biometric data of at least one polysomnography testee measured by using the EEG sensor for measuring brain waves, the EOG sensor for measuring eye movement, the EMG sensor for measuring body movement, the EKG sensor for measuring heartbeat, the PPG sensor for measuring oxygen saturation and heart rate, the thermistor and flow sensor for measuring breathing, the chest belt and abdomen belt for measuring abdominal and chest movement, and the microphone for measuring snoring. Further, the polysomnography device may generate polysomnography result information based on the biometric data.

The sleep quality scoring device 100 may store the polysomnography result information received from the polysomnography device in a database (not shown). Further, the database may store therein the polysomnography result information and information about distribution models and scoring functions to be described later.

Herein, the polysomnography result information stored in the database may be a collection of polysomnography results generated for a plurality of polysomnography testees, respectively. The polysomnography result information may be generated from the polysomnography device and transmitted to the database. Alternatively, the polysomnography result information may be processed in another database and stored in the form of a file. In this case, the database may receive polysomnography result information previously generated from another database instead of the polysomnography device.

The database may be located outside the sleep quality scoring device 100 as well as inside the sleep quality scoring device 100. In this case, the sleep quality scoring device 100 may be connected to the external database in a wired or wireless manner and may transmit and receive data or information.

The scoring function generation unit 250 may generate distribution models for respective components of polysomnography based on the polysomnography result information about the plurality of polysomnography testees, and may generate scoring functions for the respective distribution models.

Herein, the components of polysomnography may include sleep time-related components and breathing event-related components. The sleep time-related components may include total sleep time, total number of arousals, total arousal time and sleep latency, and the breathing event-related components may include the number of central sleep apneas, the number of obstructive sleep apneas, the number of hypopneas and the number of respiratory effort related arousals.

More specifically, the total sleep time among the sleep time-related components may be determined by sleep time of a polysomnography testee, and the total arousal time (Wake After Sleep Onset (WASO)) and the total number of arousals (# of wake) may be determined by the number and time of arousals of a polysomnography testee during sleep. Also, the sleep latency may refer to the amount of time it takes a polysomnography testee to fall asleep after the polysomnography testee lies in bed.

The number of central sleep apneas (Central Apnea) and the number of obstructive sleep apneas (Obstructive Apnea) among the breathing event-related components may be determined by a case where respiratory effort is lost due to any problem of the respiratory system when apnea lasts for 10 seconds or more during sleep and a case where the upper airway is narrowed and apnea occurs in spite of respiratory effort, respectively. Also, the number of hypopneas may be determined by the number of cases where the respiratory rate is rarely changed, but the depth of breathing is shallow and the tidal volume decreases. The number of respiratory effort related arousals (RERA) may refer to the number of arousals during sleep caused by an increase in respiratory effort.

The distribution models may be generated for the respective components of polysomnography by the scoring function generation unit 250. More specifically, the distribution models may be generated for the respective sleep time-related components, such as the total sleep time, the total number of arousals, the total arousal time and the sleep latency, among the components of polysomnography, and may be generated for the respective breathing event-related components, such as the number of central sleep apneas, the number of obstructive sleep apneas, the number of hypopneas and the number of respiratory effort related arousals.

That is, the distribution models be generated for the respective components based on the collected polysomnography result information. To this end, polysomnography result information about a plurality of testees stored in the database may be used. If a distribution table is generated from scores for the respective components of polysomnography measured from the plurality of polysomnography testees, it is possible to determine whether the scores are within normal values for the respective components of polysomnography according to the degree of distribution. That is, the scoring function generation unit 250 may generate histograms for the respective components from the polysomnography result information measured from the plurality of polysomnography testees, and may generate distribution models based on the histograms for the respective components.

For example, if most of the testees are distributed in a specific sleep time range (for example, 300 minutes to 450 minutes), a distribution model for the total sleep time may be generated by applying this distribution so that a value for the corresponding time range is high. For another example, values for the total arousal time, the total number of arousals and the sleep latency are low in most of the testees with normal sleep health, and, thus, a distribution model may be generated to have a high degree of distribution for a lower value. Meanwhile, most of the testees with normal sleep health show no or a lower number of breathing-related events, and, thus, a distribution model may be generated to have a high degree of distribution for a lower number of breathing-related events.

Also, the scoring function generation unit 250 may generate different distribution models depending on the age group or disease group of the polysomnography testees. More specifically, different distribution models for respective age groups or disease groups of the testees may be used in order to calculate a sleep quality score more accurately in consideration of physical characteristics or disease features of the testees. More specifically, a distribution model may be set by using information input from a polysomnography testee or a polysomnography tester. For example, the scoring function generation unit 250 may generate distribution models appropriate for the respective genders or age groups of the polysomnography testees by using polysomnography results measured from the respective genders or age groups. Further, the scoring function generation unit 250 may a generate different distribution model for each of a hypertension patient and a patient who takes sleeping pills, and information about gender, age, disease, etc. may be based on polysomnography results derived by filtering the polysomnography results stored in the database.

The scoring function generation unit 250 may generate scoring functions for the generated distribution models, respectively. The scoring functions generated for the respective distribution models may be functions for conversion into scores corresponding to the respective components based on the generated distribution models. That is, the scoring functions are generated for the respective components of polysomnography, and when sleep time information or event occurrence information to each corresponding components is input, a scoring function may convert a value for the sleep time information or event occurrence information into a score depending on the position in the distribution models.

The scoring functions may basically use a Gaussian distribution, but all the components cannot have negative values, and, thus, an additional score conversion process is needed. The scoring functions for the sleep time-related components, particularly the total arousal time, the total number of arousals and the sleep latency, may be set to produce the highest scores when the total arousal time, the total number of arousals and the sleep latency have lower values than the highest scores.

Also, the scoring functions for the breathing event-related components may be set to calculate a standard deviation when the mean of the Gaussian distribution is 0.

As described above, the sleep quality scoring device 100 may determine sleep-related symptoms, such as OSA, CSA and mixed sleep apnea, of the subject by using the transceiver 210.

While the sleep quality scoring device 100 determines sleep-related symptoms such as OSA, CSA and mixed sleep apnea, the information derivation unit 220 may identify the number of CSAs, the number of OSAs, the number of hypopneas and the number of respiratory effort related arousals.

Therefore, the sleep quality scoring device 100 can precisely determine sleep-related symptoms of the subject and analyze the causes as in polysomnography without performing polysomnography.

Further, the sleep quality scoring device 100 may identify a sleep start time and a sleep end time of the subject by means of the transceiver 210, and may also identify an arousal time and an arousal duration during sleep.

Then, the information derivation unit 220 may derive a total sleep time based on a difference between the sleep start time and the sleep end time. Also, the information derivation unit 220 may derive a total number of arousals and a total arousal time based on the arousal time and arousal duration during sleep. Further, the information derivation unit 220 may derive a sleep latency by analyzing motions based on the sleep start time and radar signals.

That is, the sleep time information derived by the information derivation unit 220 may include a component corresponding to at least one of the total sleep time, the total number of arousals and the sleep latency among the components of polysomnography, and the event occurrence information derived by the information derivation unit 220 may include a component corresponding to at least one of the number of CSAs, the number of OSAs, the number of hypopneas and the number of respiratory effort related arousals among the components of polysomnography.

The component score calculation unit 260 may calculate scores for the respective components by applying the information derived by the information derivation unit 220 to the scoring functions. More specifically, the component score calculation unit 260 may apply a scoring function to a measurement value generated for the subject by the information derivation unit 220 and thus may derive a score for each component of polysomnography in the range of 0 to 100.

The sleep quality score calculation unit 270 may calculate a sleep quality score of the subject based on the scores for the respective components calculated by the component score calculation unit 260. More specifically, the sleep quality score calculation unit 270 may derive a sleep quality score by averaging all the calculated scores for the respective components of polysomnography.

The sleep quality score may be displayed on a separate display device through an output device of the sleep quality scoring device 100 or may be provided to another device connected thereto.

Also, the sleep quality score calculation unit 270 may give weightings to the calculated scores for the respective components based on feedback information input from the subject and calculate a weighted sleep quality score. Herein, the feedback information may correspond to information generated based on feedback on last sleep input into the sleep quality scoring device 100 by the subject after sleep.

More specifically, the feedback information may include information input by the subject in response to a question about a sleep satisfaction level in sleep tests performed daily or intermittently by the sleep quality scoring device 100. The sleep quality scoring device 100 may generate weightings by analyzing a correlation between values for components of the respective sleep test items calculated on a date when feedback is to be received and a feedback satisfaction level and identifying conformity between variations in feedback and variations in values for the respective components. Herein, a sleep test entry given a higher weighting may be more sensitive in assessing the sleep quality of the subject. Therefore, the weighted sleep quality score is more suitable to represent the state of the subject and can measure the sleep quality of the subject more accurately.

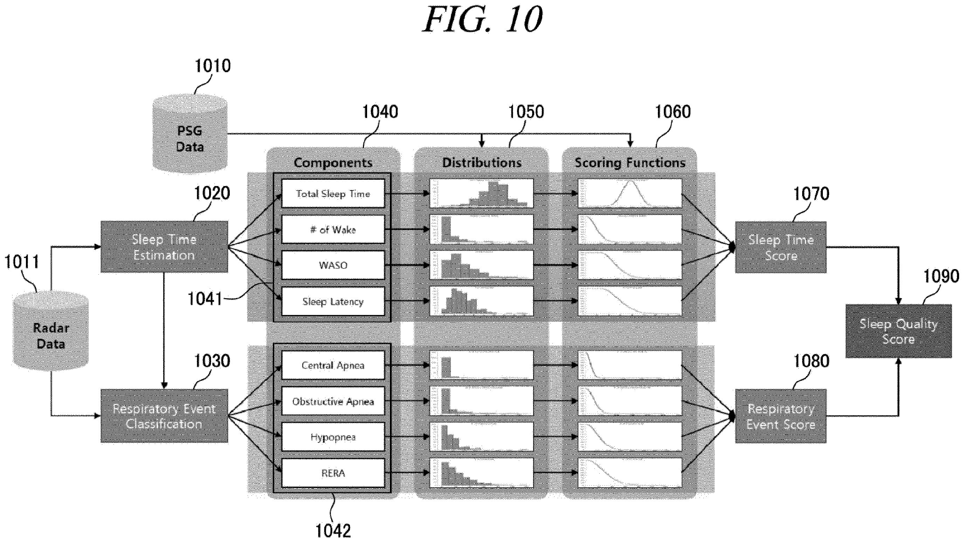

FIG. 10 is a diagram for explaining a process of scoring sleep quality according to an embodiment of the present disclosure.

Referring to FIG. 10, it can be seen that the sleep quality scoring device generates distribution models 1050 and scoring functions 1060 based on data extracted from a database 1010, and performs sleep time estimation 1020 and respiratory event classification 1030 based on information extracted from a database 1011 in which radar signals are stored. Also, it can be seen that the sleep quality scoring device 100 generates information about each of sleep time-related components 1041 through the sleep time estimation 1020 and information about each of breathing event-related components 1042 through the respiratory event classification 1030. Further, it can be seen that the sleep quality scoring device 100 calculates scores 1070 for the respective sleep time-related components and scores 1080 for the respective breathing event-related components 1042 by applying the distribution models 1050 and the scoring functions 1060 for respective components 1040 and calculates a sleep quality score 1090 by aggregating the calculated scores.

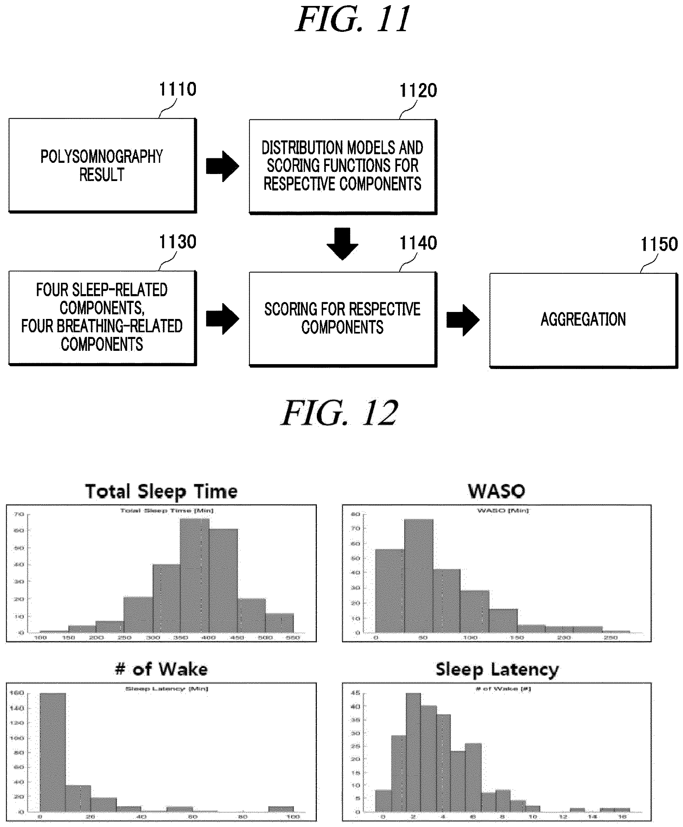

Referring to FIG. 11, it can be seen that a sleep quality score is derived by generating distribution models and scoring functions for the respective components (1120) from a polysomnography result 1110, calculating scores for the respective components (1140) by applying the scoring functions to sleep-related components and breathing-related components (1130), and aggregating the calculated scores (1150).

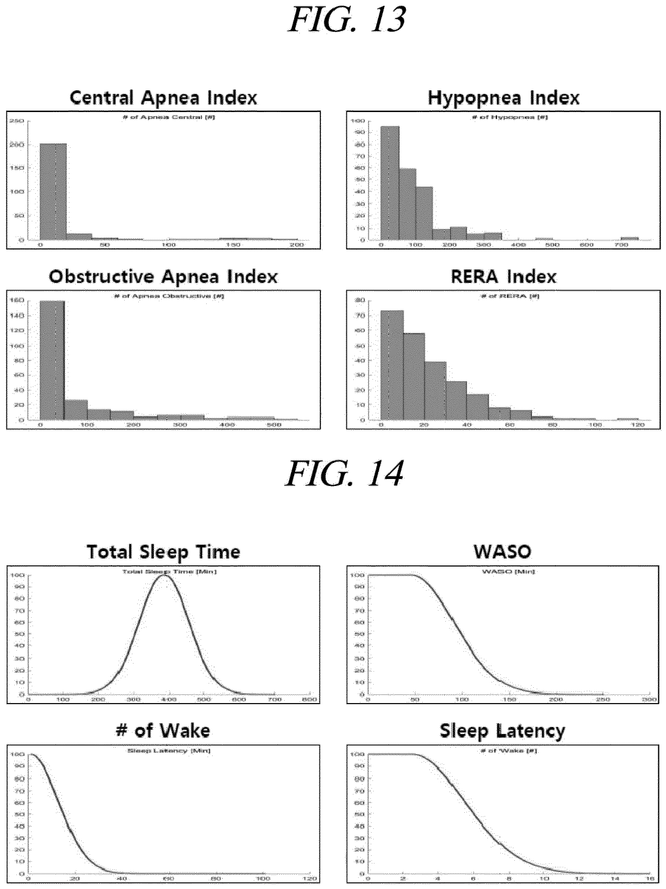

FIG. 12 and FIG. 13 show graphs for explaining distribution models according to an embodiment of the present disclosure. FIG. 12 shows examples of distribution models generated for sleep time-related components, and FIG. 13 shows examples of distribution models generated for breathing event-related components. It can be seen from FIG. 12 that the distribution model for the total sleep time shows a high degree of distribution in the range of 300 minutes to 450 minutes, whereas the distribution model for the total arousal time (WASO) shows a high degree of distribution in the range of 0 minutes to 100 minutes.

**FIG. 13** shows examples of distribution models for breathing event-related components, and it can be seen from **FIG. 13** that the degree of distribution is higher when the frequency of each breathing event is lower.

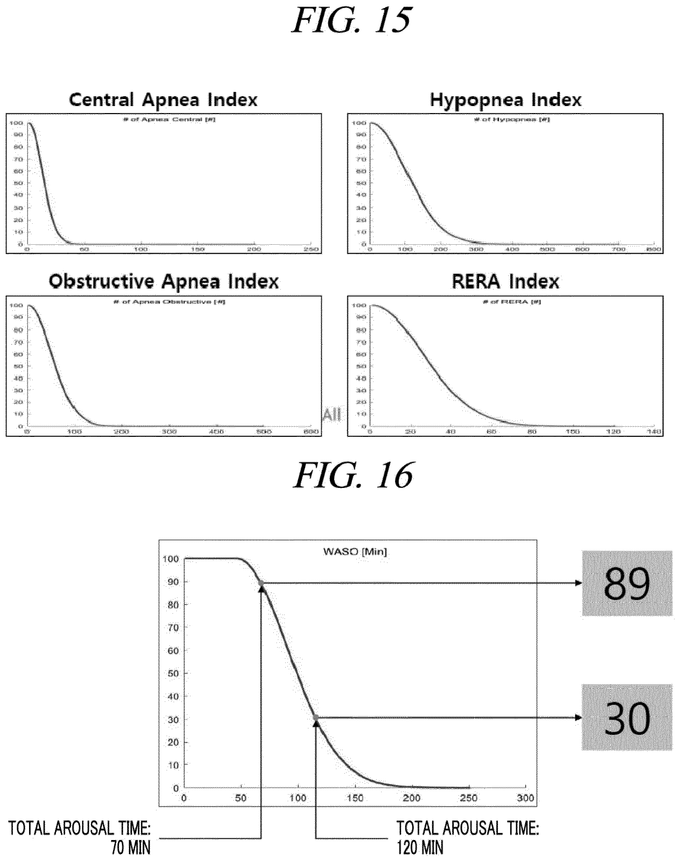

**FIG. 14** and **FIG. 15** show graphs for explaining scoring functions according to an embodiment of the present disclosure.

**FIG. 14** shows examples of scoring functions for the sleep time-related components, and **FIG. 15** shows examples of scoring functions for the breathing event-related components. More specifically, it can be seen from **FIG. 14** that the scoring functions are determined to assign a higher score to a range with a higher degree of distribution in the distribution models based on the distribution models shown in **FIG. 12.** However, the scoring functions for the total arousal time (WASO), the total number of arousals (# of wake) and the sleep latency may be set to produce the highest scores when the total arousal time, the total number of arousals and the sleep latency have lower values than the highest scores.

It can be seen from **FIG. 15** that the scoring functions are determined to assign a higher score to a lower frequency based on the distribution models shown in **FIG. 13.** Herein, it can be assumed that the scoring functions shown in **FIG. 15** recalculate a standard deviation when the mean of the Gaussian distribution is 0 and a symmetric distribution is present in the negative region (second quadrant).

**FIG. 16** is a graph for explaining a process of calculating a score for each element by inputting a measurement value into the scoring function of **FIG. 14.**

More specifically, **FIG. 16** shows an example of the scoring function for the total arousal time among the scoring functions shown in **FIG. 14.** The component score calculation unit may calculate a score for the total arousal time by substituting the total arousal time of the subject into the corresponding scoring function. **FIG. 16** illustrates an example where the scoring function is generated to calculate a score of 89 when the total arousal time of the subject is 70 minutes and a score of 30 when the total arousal time of the subject is 120 minutes. **FIG. 8** illustrates a part of a process of calculating scores through the scoring functions for the respective components, and scores for the components other than the total arousal time shown in **FIG. 8** may also be calculated as shown in **FIG. 16.**

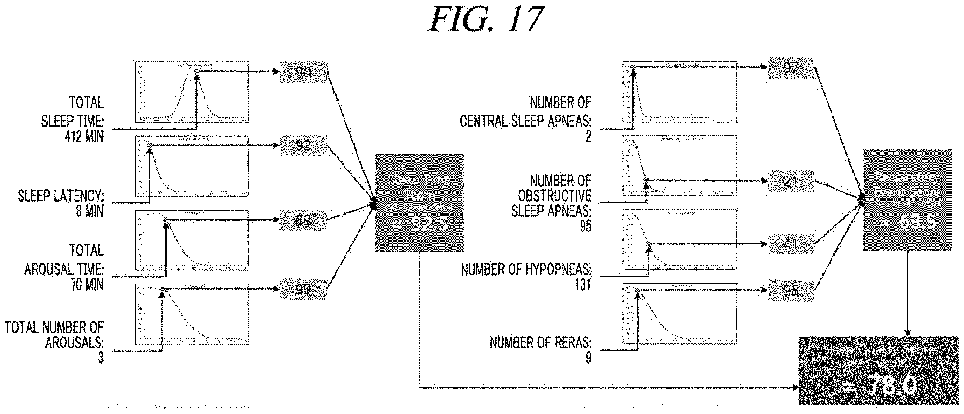

**FIG. 17** is provided to explain a process of calculating a sleep quality score based on scores calculated for respective components of polysomnography according to an embodiment of the present disclosure. Referring to **FIG. 17,** it can be seen that the sleep quality scoring device calculates a sleep quality score based on the mean of the scores calculated for the respective components.

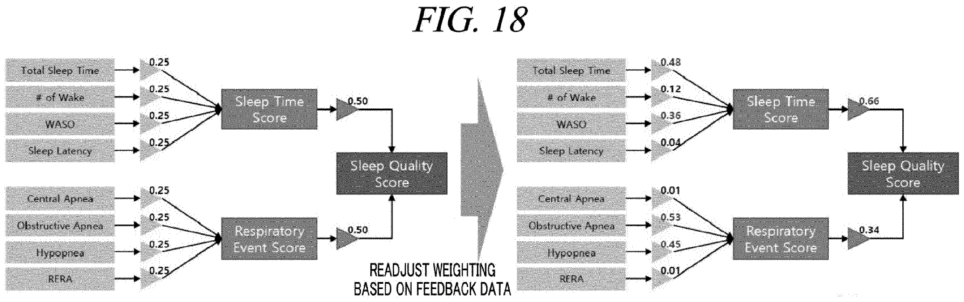

**FIG. 18** is provided to explain a process of calculating a sleep quality score by giving weightings to scores calculated for respective components of polysomnography according to an embodiment of the present disclosure. The left diagram in **FIG. 18** illustrates an example of calculating a sleep quality score by giving an equal weighting (0.25) to the scores calculated for the respective components of polysomnography. The right diagram in **FIG. 18** illustrates an example of calculating a sleep quality score by readjusting weightings based on feedback data and giving different weightings to the scores calculated for the respective components of polysomnography.

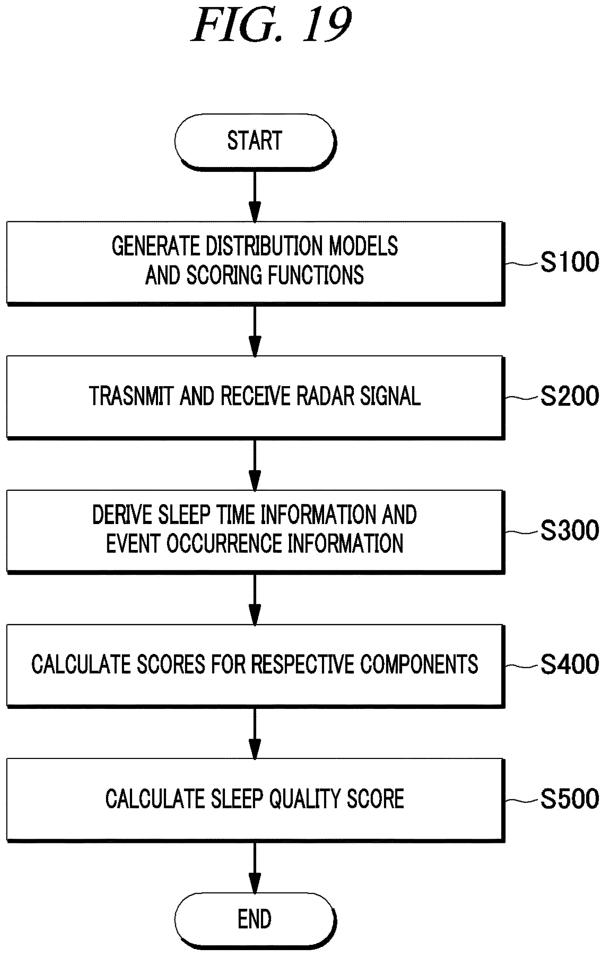

**FIG. 19** is a flowchart showing a method for scoring sleep quality according to an embodiment of the present disclosure. Referring to **FIG. 19,** the method for scoring sleep quality according to an embodiment of the present disclosure may include: a scoring function generating process S100 of generating distribution models for respective components of polysomnography based on polysomnography result information about a plurality of polysomnography testees and generating scoring functions for the respective distribution models; a transceiving process S200 of transmitting a radar signal toward a subject and receiving the radar signal reflected from the subject; a deriving process S300 of deriving sleep time information about the subject and event occurrence information for a plurality of sleep items based on the radar signal; a component score calculating process S400 of calculating scores for the respective components by applying the sleep time information and the event occurrence information to the scoring functions; and a process S500 of calculating a sleep quality score of the subject based on the calculated scores for the respective components.

The method for scoring sleep quality may be divided into additional processes or combined into fewer processes according to the embodiment described above with reference to **FIG. 1** to **FIG. 18.** In addition, some of the processes may be omitted and the sequence of the processes may be changed if necessary.

The method for scoring sleep quality can be implemented in a computer program stored in a medium to be executed by a computer or a storage medium including instructions codes executable by a computer. Also, the method for scoring sleep quality can be implemented in a computer program stored in a medium to be executed by a computer.

A computer-readable medium can be any usable medium which can be accessed by the computer and includes all volatile/non-volatile and removable/non-removable media. Further, the computer-readable medium may include all computer storage and communication media. The computer storage medium includes all volatile/non-volatile and removable/non-removable media embodied by a certain method or technology for storing information such as computer-readable instruction code, a data structure, a program module or other data. The communication medium typically includes the computer-readable instruction code, the data structure, the program module, or other data of a modulated data signal such as a carrier wave, or other transmission mechanism, and includes a certain information transmission medium.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

**Claims**

1. A device for scoring sleep quality, comprising:

> a scoring function generation unit configured to generate distribution model for at least one of components of polysomnography based on polysomnography result information about a plurality of polysomnography testees and generate scoring function for the distribution model;
> a transceiver configured to transmit a radar signal toward a subject and receive the radar signal reflected from the subject;
> an information derivation unit configured to derive sleep time information about the subject and event occurrence information for a plurality of sleep items based on the radar signal;
> a component score calculation unit configured to calculate score for the at least one of components by applying the sleep time information and the event occurrence information to the scoring function; and
> a sleep quality calculation unit configured to calculate a sleep quality score of the subject based on the calculated score for the at least one of components.

2. The device for scoring sleep quality of Claim 1,
   wherein the sleep time information of the subject includes a component corresponding to at least one of a total sleep time, a total number of arousals and a sleep latency among the components of polysomnography.

3. The device for scoring sleep quality of Claim 1 or 2,
   wherein the event occurrence information of the subject includes a component corresponding to at least one of a number of central sleep apneas, a number of obstructive sleep apneas, a number of hypopneas and a number of respiratory effort related arousals among the components of polysomnography.

4. The device for scoring sleep quality of any one of Claims 1 to 3,
   wherein the scoring function generation unit is further configured to generate histograms for respective components from the polysomnography result information measured from the plurality of polysomnography testees, and generate the distribution model based on the histograms for the respective components.

5. The device for scoring sleep quality of any one of Claims 1 to 4,
   wherein the scoring function generation unit is further configured to generate different distribution models depending on at least one of an age group and a disease group of the polysomnography testees.

6. The device for scoring sleep quality of any one of Claims 1 to 5,
   wherein the scoring function is generated for the distribution model and converts a value for the sleep time information or the event occurrence information input to each corresponding component into a score depending on a position in the distribution model.

7. The device for scoring sleep quality of any one of Claims 1 to 6,

> wherein the sleep quality score calculation unit is further configured to give weightings to the score and calculate a weighted sleep quality score, and
> the weightings are calculated based on feedback information input from the subject.

8. A method for scoring sleep quality using a sleep quality scoring device, comprising:

generating distribution model for at least one of component of polysomnography based on polysomnography result information about a plurality of polysomnography testees and generating scoring function for the distribution model;

transmitting a radar signal toward a subject and receiving the radar signal reflected from the subject;

deriving sleep time information about the subject and event occurrence information for a plurality of sleep items based on the radar signal;

calculating score for the at least one of components by applying the sleep time information and the event occurrence information to the scoring function; and

calculating a sleep quality score of the subject based on the calculated score for the at least one of components.

9. The method for scoring sleep quality of Claim 8,
wherein the sleep time information of the subject includes a component corresponding to at least one of a total sleep time, a total number of arousals and a sleep latency among the components of polysomnography.

10. The method for scoring sleep quality of Claim 8 or 9,
wherein the event occurrence information of the subject includes a component corresponding to at least one of a number of central sleep apneas, a number of obstructive sleep apneas, a number of hypopneas and a number of respiratory effort related arousals among the components of polysomnography.

11. The method for scoring sleep quality of any one of Claims 8 to 10,
wherein the generating scoring function includes:

generating histograms for respective components from the polysomnography result information measured from the plurality of polysomnography testees, and

generating the distribution model based on the histograms for the respective components.

12. The method for scoring sleep quality of any one of Claims 8 to 11,
wherein the generating scoring function includes:
generating different distribution models depending on at least one of an age group and a disease group of the polysomnography testees.

13. The method for scoring sleep quality of any one of Claims 8 to 12,
wherein the scoring function is generated for the distribution model and converts a value for the sleep time information or the event occurrence information input to each corresponding component into a score depending on a position in the distribution model.

14. The method for scoring sleep quality of any one of Claims 8 to 13,
wherein the calculating the sleep quality score includes:

giving weightings to the score and calculating a weighted sleep quality score, and

wherein the weightings are calculated based on feedback information input from the subject.

15. A non-transitory computer-readable storage medium that stores a sequence of instructions for scoring sleep quality,
wherein the sequence of instructions, when executed by a computing device, causes a computing device to:

generate distribution model for at least one of components of polysomnography based on polysomnography result information about a plurality of polysomnography testees and generate scoring function for the respective distribution model;

transmit a radar signal toward a subject and receive the radar signal reflected from the subject;

derive sleep time information about the subject and event occurrence information for a plurality of sleep items based on the radar signal;

calculate score for the at least one of components by applying the sleep time information and the event occurrence information to the scoring function; and

calculate a sleep quality score of the subject based on the calculated score for the at least one of components.

## FIG. 1

<u>1</u>

## FIG. 2

## FIG. 3

## FIG. 4A

EP 4 241 657 A1

*FIG. 4B*

*FIG. 5*

*FIG. 6*

# FIG. 7A

| | CLASSIFICATION | DETECTION ENTRY | L | F | T | b0 | b1 | b2 | b3 | b4 |
|---|---|---|---|---|---|---|---|---|---|---|
| | | | 721 | 722 | 723 | 731 | 732 | 733 | 734 | 735 |
| | | | | | | | | | | |
| 711 | APNEA | CENTRAL ~711a | 30 | 1.5 | 17.5 | −3.21 | 1.28 | −1.38 | 0.05 | −0.05 |
| | | MIXED ~711b | 20 | 1.75 | 7.5 | −11.05 | 0.00 | 0.02 | 0.96 | −1.02 |
| | | OBSTRUCTIVE ~711c | 30 | 1.75 | 5 | 62.68 | −0.07 | −0.06 | −0.19 | 1.30 |
| 712 | HYPOPNEA | CENTRAL | 5 | 0.5 | 0.3 | 0.00 | 0.00 | 0.00 | 0.00 | 0.00 |
| | | MIXED | 5 | 0.5 | 12.5 | −0.02 | 0.00 | 0.97 | 0.00 | 0.00 |
| | | OBSTRUCTIVE | 10 | 1.75 | 7.5 | 14.05 | −0.40 | 0.87 | −0.66 | 0.85 |
| 713 | TEMPORARY AROUSAL | LM(LIMB MOVEMENT-RELATED AROUSAL) | 10 | 1.75 | 7.5 | −2.51 | −0.09 | 0.10 | 1.01 | −1.14 |
| | | RERA(RESPIRATORY EFFORT-RELATED AROUSAL) | 40 | 1.75 | 7.5 | −1.45 | 0.07 | −0.07 | −0.03 | −0.07 |
| | | Resp(APNEA-RELATED AROUSAL/ | 30 | 1.75 | 5 | 29.81 | −0.03 | 0.01 | −0.03 | 1.15 |
| | | HYPOPNEA-RELATED AROUSAL) | 60 | 1.75 | 1 | 3.10 | 0.00 | −0.01 | −0.02 | 0.02 |
| | | Snore(SNORING-RELATED AROUSAL) | 20 | 1.75 | 0.5 | −13.56 | 0.00 | −0.01 | 0.01 | 0.01 |
| | | Spontaneous(SPONTANEOUS AROUSAL) | | | | | | | | |

(710 — DETECTION ENTRY column; 720 spans 721, 722, 723; 730 spans 731, 732, 733, 734, 735)

# FIG. 7B

EP 4 241 657 A1

| # Target Event | # of Events | All | Headboard V30 | Headboard T0 | Ceiling V30 | Ceiling T0 | Ceiling T1 | Optimized |
|---|---|---|---|---|---|---|---|---|
| 1 Apnea | 0 | 0.0000 | 0.0000 | 0.0000 | 0.0000 | 0.0000 | 0.0000 | 0.0000 |
| 2 Apnea Central | 811 | 0.6452 | 0.9250 | 0.9094 | 0.5603 | 0.5997 | 0.9206 | 0.7946 |
| 3 Apnea Mixed | 2691 | 0.8912 | 0.9921 | 0.9942 | 0.7986 | 0.8520 | 0.9593 | 0.9551 |
| 4 Apnea Obstructive | 18364 | 0.6587 | 0.8896 | 0.7320 | 0.7000 | 0.7247 | 0.9316 | 0.7702 |
| 5 Hypopnea | 0 | 0.0000 | 0.0000 | 0.0000 | 0.0000 | 0.0000 | 0.0000 | 0.0000 |
| 6 Hypopnea Central | 0 | 0.0000 | 0.0000 | 0.0000 | 0.0000 | 0.0000 | 0.0000 | 0.0000 |
| 7 Hypopnea Mixed | 8 | 0.2336 | 0.3791 | 0.4355 | 0.5125 | 0.4410 | 0.0000 | 0.4470 |
| 8 Hypopnea Obstructive | 23440 | 0.4279 | 0.7559 | 0.5517 | 0.4737 | 0.4647 | 0.7707 | 0.5960 |
| 9 R | 21590 | 0.3776 | 0.6377 | 0.5691 | 0.6447 | 0.6087 | 0.8119 | 0.6429 |
| 10 W | 32167 | 0.2361 | 0.6709 | 0.6383 | 0.4562 | 0.5312 | 0.7526 | 0.5751 |
| 11 N1 | 27734 | 0.7171 | 0.9071 | 0.7672 | 0.7675 | 0.8281 | 0.9703 | 0.8282 |
| 12 N2 | 57991 | 0.3689 | 0.8135 | 0.7419 | 0.7526 | 0.7882 | 0.7786 | 0.7763 |
| 13 N3 | 20328 | 0.5608 | 0.5793 | 0.5245 | 0.6616 | 0.6096 | 0.7561 | 0.6264 |
| 14 S4 | 0 | 0.0000 | 0.0000 | 0.0000 | 0.0000 | 0.0000 | 0.0000 | 0.0000 |
| 15 LM Arousal | 1325 | 0.4324 | 0.4524 | 0.2598 | 0.6012 | 0.6129 | 0.8386 | 0.6215 |
| 16 RERA | 4574 | 0.3917 | 0.6498 | 0.5740 | 0.6109 | 0.5106 | 0.8326 | 0.6247 |
| 17 Resp Arousal | 27773 | 0.7570 | 0.9488 | 0.7992 | 0.7564 | 0.6734 | 0.9564 | 0.8175 |
| 18 Snore Arousal | 3040 | 0.3355 | 0.5166 | 0.5978 | 0.6015 | 0.5615 | 0.6895 | 0.5845 |
| 19 Spontaneous Arousal | 2987 | 0.4748 | 0.5820 | 0.4448 | 0.6517 | 0.6628 | 0.8316 | 0.6172 |
| 20 Apnea All | 21866 | 0.7754 | 0.9476 | 0.8175 | 0.7441 | 0.7677 | 0.9294 | 0.8254 |
| 21 Hypopnea All | 23448 | 0.4279 | 0.7560 | 0.5519 | 0.4739 | 0.4647 | 0.7707 | 0.5962 |
| 22 Araousal All | 39699 | 0.7089 | 0.9184 | 0.7948 | 0.7683 | 0.7016 | 0.9720 | 0.8143 |
| 23 Apnea + Hypopnea | 45314 | 0.6759 | 0.8858 | 0.7149 | 0.7358 | 0.6660 | 0.9317 | 0.7730 |
| 24 Apnea + Hypopnea + Wake | 77481 | 0.6637 | 0.9431 | 0.7610 | 0.7514 | 0.7336 | 0.8809 | 0.8058 |
| 25 Apnea + Hypopnea + Araousal | 85013 | 0.7263 | 0.9389 | 0.7665 | 0.7676 | 0.7191 | 0.9601 | 0.8164 |
| 26 Apnea + Hypopnea + Araousal + Wake | 117180 | 0.7246 | 0.9558 | 0.7830 | 0.7977 | 0.7735 | 0.9403 | 0.8386 |
| 27 Central | 811 | 0.6452 | 0.9250 | 0.9094 | 0.5603 | 0.5997 | 0.9206 | 0.7946 |
| 28 Mixed | 2699 | 0.8912 | 0.9921 | 0.9943 | 0.7986 | 0.8526 | 0.9593 | 0.9552 |
| 29 Obstuctive | 41804 | 0.6133 | 0.8554 | 0.6973 | 0.6794 | 0.5981 | 0.9293 | 0.7327 |
| 30 Apnea + Hypopnea + RERA | 49888 | 0.6694 | 0.8858 | 0.7138 | 0.7365 | 0.6760 | 0.9362 | 0.7734 |
| 31 Position-Supine | 117543 | 0.2923 | 0.7593 | 0.6618 | 0.7712 | 0.7783 | 0.8072 | 0.7617 |
| 32 Position-Prone | 141 | 0.2326 | 0.2888 | 0.3536 | 0.3237 | 0.3579 | 0.7852 | 0.3567 |
| 33 Position-Left | 14134 | 0.1983 | 0.2682 | 0.3400 | 0.4825 | 0.5559 | 0.6829 | 0.4540 |
| 34 Position-Right | 22005 | 0.3387 | 0.5968 | 0.5981 | 0.5802 | 0.4313 | 0.5796 | 0.5658 |
| 35 Position-Upright | 0 | 0.0000 | 0.0000 | 0.0000 | 0.0000 | 0.0000 | 0.0000 | 0.0000 |
| 36 Position-Left + Position-Right | 36139 | 0.3243 | 0.5077 | 0.4350 | 0.6210 | 0.5223 | 0.6104 | 0.5398 |
| Population | | 219 | 43 | 43 | 57 | 60 | 24 | 227 |

740

750
751
752
753
754

## FIG. 8A

## FIG. 8B

## FIG. 8C

## FIG. 9

| | | Value | AD | Control | Prediction |
|---|---|---|---|---|---|
| 910 { | 911 — TST | 183 —921 | 0.8692 —931 | 0.1308 —941 | AD —951 |
| | 912 — AHI | 10.49 —922 | 0.2179 —932 | 0.7821 —942 | Control —952 |
| | 913 — RDI | 14.43 —923 | 0.9801 —933 | 0.0199 —943 | AD —953 |

*FIG. 10*

# FIG. 11

```
          1110                              1120
   ┌──────────────────┐          ┌──────────────────────┐
   │                  │          │ DISTRIBUTION MODELS AND │
   │  POLYSOMNOGRAPHY │   ──▶    │ SCORING FUNCTIONS FOR   │
   │     RESULT       │          │ RESPECTIVE COMPONENTS   │
   └──────────────────┘          └──────────────────────┘

          1130                              1140                        1150
   ┌──────────────────┐          ┌──────────────────────┐     ┌──────────────────┐
   │ FOUR SLEEP-RELATED│          │                      │     │                  │
   │    COMPONENTS,    │   ──▶    │ SCORING FOR RESPECTIVE│ ──▶ │   AGGREGATION    │
   │ FOUR BREATHING-RELATED│      │     COMPONENTS        │     │                  │
   │    COMPONENTS     │          │                      │     │                  │
   └──────────────────┘          └──────────────────────┘     └──────────────────┘
```

# FIG. 12

Total Sleep Time

WASO

# of Wake

Sleep Latency

# FIG. 13

### Central Apnea Index

### Hypopnea Index

### Obstructive Apnea Index

### RERA Index

# FIG. 14

### Total Sleep Time

### WASO

### # of Wake

### Sleep Latency

## FIG. 15

**Central Apnea Index**

# of Apnea Central [#]

**Hypopnea Index**

# of Hypopnea [#]

**Obstructive Apnea Index**

# of Apnea Obstructive [#]

**RERA Index**

# of RERA [#]

## FIG. 16

WASO [Min]

89

30

TOTAL AROUSAL TIME:
70 MIN

TOTAL AROUSAL TIME:
120 MIN

*FIG. 17*

FIG. 18

# *FIG. 19*

START

↓

| GENERATE DISTRIBUTION MODELS AND SCORING FUNCTIONS | — S100 |

↓

| TRASNMIT AND RECEIVE RADAR SIGNAL | — S200 |

↓

| DERIVE SLEEP TIME INFORMATION AND EVENT OCCURRENCE INFORMATION | — S300 |

↓

| CALCULATE SCORES FOR RESPECTIVE COMPONENTS | — S400 |

↓

| CALCULATE SLEEP QUALITY SCORE | — S500 |

↓

END

Europäisches
Patentamt

European
Patent Office

Office européen
des brevets

## EUROPEAN SEARCH REPORT

Application Number

**EP 23 16 0941**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/220247 A1 (RESMED SENSOR TECH LTD [IE]) 4 November 2021 (2021-11-04) <br> * figures 1, 8 * <br> * paragraphs [0054] – [0060], [0075], [0144], [0195] – [0198] * | 1-15 | INV. <br> A61B5/00 <br> A61B5/0507 <br> G16H50/30 |
| X | US 2020/367810 A1 (SHOULDICE REDMOND [IE] ET AL) 26 November 2020 (2020-11-26) <br> * paragraphs [0194], [0219], [0247] * | 1,8,15 | |
| X | US 2020/388287 A1 (ANUSHIRAVANI RAMIN [US] ET AL) 10 December 2020 (2020-12-10) <br> * figure 11 * <br> * paragraphs [0215], [0137] * | 1,8,15 | |

TECHNICAL FIELDS
SEARCHED      (IPC)

A61B
G16H

The present search report has been drawn up for all claims

| Place of search | Date of completion of the search | Examiner |
|---|---|---|
| The Hague | 11 July 2023 | Schwenke, Stephanie |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...............................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 16 0941

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2023

| Patent document cited in search report | | Publication date | Patent family member(s) | | Publication date |
|---|---|---|---|---|---|
| WO 2021220247 | A1 | 04-11-2021 | AU | 2021265344 A1 | 08-12-2022 |
| | | | CN | 115836358 A | 21-03-2023 |
| | | | EP | 4143849 A1 | 08-03-2023 |
| | | | US | 2023173221 A1 | 08-06-2023 |
| | | | WO | 2021220247 A1 | 04-11-2021 |
| US 2020367810 | A1 | 26-11-2020 | CN | 111655125 A | 11-09-2020 |
| | | | EP | 3727134 A1 | 28-10-2020 |
| | | | JP | 7202385 B2 | 11-01-2023 |
| | | | JP | 2021508279 A | 04-03-2021 |
| | | | KR | 20200104341 A | 03-09-2020 |
| | | | US | 2020367810 A1 | 26-11-2020 |
| | | | WO | 2019122412 A1 | 27-06-2019 |
| US 2020388287 | A1 | 10-12-2020 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220030033
- KR 1020230021306
- KR 102321991
- KR 1020180077453